# EUROPEAN PATENT SPECIFICATION

(11) **EP 3 394 267 B1**
(45) Date of publication and mention of the grant of the patent: **21.10.2020**
(21) Application number: 16816292.3
(22) Date of filing: 21.12.2016
(51) Int. Cl.: C12N 15/63, C12N 15/85, C12N 5/071

(54) **PROGRAMMABLE DIFFERENTIATION CONTROL NETWORK**
PROGRAMMIERBARES DIFFERENZIERUNGSSTEUERNETZWERK
RÉSEAU DE COMMANDE DE DIFFÉRENCIATION PROGRAMMABLE

(30) Priority: 24.12.2015 EP 15202677
(43) Date of publication of application: 31.10.2018
(73) Proprietor: ETH Zurich, 8092 Zurich (CH)
(72) Inventor: FUSSENEGGER, Martin, 5506 Mägenwil (CH); SAXENA, Pratik, 4058 Basel (CH); ZULEWSKI, Henryk, 4102 Binningen (CH)
(74) Representative: Latscha Schöllhorn Partner AG
(86) International application number: PCT/EP2016/082121
(87) International publication number: WO 2017/108915

(56) References cited:
- WO-A1-2005/021766
- WO-A2-2010/022395
- WO-A2-2014/117945
- SIMON AUSLÄNDER ET AL: "From gene switches to mammalian designer cells: present and future prospects", TRENDS IN BIOTECHNOLOGY, vol. 31, no. 3, 1 March 2013 (2013-03-01), pages 155-168, XP055117371, ISSN: 0167-7799, DOI: 10.1016/j.tibtech.2012.11.006
- AUSLÄNDER DAVID ET AL: "A Synthetic Multifunctional Mammalian pH Sensor and CO2Transgene-Control Device", MOLECULAR CELL, vol. 55, no. 3, 7 August 2014 (2014-08-07) , pages 397-408, XP029041271, ISSN: 1097-2765, DOI: 10.1016/J.MOLCEL.2014.06.007
- MING-TANG CHEN ET AL: "Artificial cell-cell communication in yeast Saccharomyces cerevisiae using signaling elements from Arabidopsis thaliana", NATURE BIOTECHNOLOGY, vol. 23, no. 12, 20 November 2005 (2005-11-20), pages 1551-1555, XP055280709, US ISSN: 1087-0156, DOI: 10.1038/nbt1162
- J. HANSEN ET AL: "Transplantation of prokaryotic two-component signaling pathways into mammalian cells", PROCEEDINGS OF THE NATIONAL ACADEMY OF SCIENCES, vol. 111, no. 44, 4 November 2014 (2014-11-04), pages 15705-15710, XP055198288, ISSN: 0027-8424, DOI: 10.1073/pnas.1406482111
- M. GITZINGER ET AL: "The food additive vanillic acid controls transgene expression in mammalian cells and mice", NUCLEIC ACIDS RESEARCH, vol. 40, no. 5, 19 December 2011 (2011-12-19), pages e37-e37, XP055241555, GB ISSN: 0305-1048, DOI: 10.1093/nar/gkr1251
- SIMON AUSLÄNDER ET AL: "Programmable single-cell mammalian biocomputers", NATURE, 3 June 2012 (2012-06-03), XP055277208, United Kingdom ISSN: 0028-0836, DOI: 10.1038/nature11149
- MINGQI XIE ET AL: "Mammalian designer cells: Engineering principles and biomedical applications", BIOTECHNOLOGY JOURNAL, vol. 10, no. 7, 26 May 2015 (2015-05-26), pages 10051018-n/a, XP055277367, DE ISSN: 1860-6768, DOI: 10.1002/biot.201400642
- BOON CHIN HENG ET AL: "G Protein-Coupled Receptors Revisited: Therapeutic Applications Inspired by Synthetic Biology", ANNUAL REVIEW OF PHARMACOLOGY AND TOXICOLOGY., vol. 54, no. 1, 6 January 2014 (2014-01-06), pages 227-249, XP055281213, US ISSN: 0362-1642, DOI: 10.1146/annurev-pharmtox-011613-135921
- PRATIK SAXENA ET AL: "A programmable synthetic lineage-control network that differentiates human IPSCs into glucose-sensitive insulin-secreting beta-like cells", NATURE COMMUNICATIONS, vol. 7, 11 April 2016 (2016-04-11), page 11247, XP055277418, United Kingdom ISSN: 2041-1723, DOI: 10.1038/ncomms11247

## Description

The present invention relates to expression system for establishing a transgenic differentiation-control network useful for controlling cell differentiation. Methods for controlling cell differentiation in a programmed manner using such expression system as well as methods for differentiating mammalian cells into pancreatic beta-like cells are also provided.

### BACKGROUND

Type 1 diabetes mellitus (T1DM) is characterized by the autoimmune destruction of insulin-producing pancreatic beta-cells, thus making patients dependent on exogenous insulin to control their blood glucose (Nathan DM. Nat Rev Endocrinol 8, 699-700 (2012); Atkinson MA, Eisenbarth GS. Lancet 358, 221-229 (2001)). Although insulin therapy has changed the prospects and survival of T1DM patients, these patients still suffer from diabetic complications arising from the lack of physiological insulin secretion and excessive glucose levels (Nathan DM. N Engl J Med 328, 16761685 (1993)). The replacement of the pancreatic beta-cells either by pancreas transplantation or by transplantation of human pancreatic islets has been shown to normalize blood glucose and even improve already existing complications of diabetes (Fioretto P, et al., N Engl J Med 339, 69-75 (1998)). Insulin independence after 5 years following islet transplantation can be achieved today in up to 55% of patients with a new modified immune suppression strategy, according to recent studies (Posselt AM, et al. Transplantation 90, 1595-1601 (2010); Turgeon NA, et al. Am J Transplant 10, 2082-2091 (2010); Shapiro AM, et al. Engl J Med 343, 230-238 (2000); Jacobs-Tulleneers-Thevissen D, et al. Diabetologia 56, 1605-1614 (2013)).

However, because of the shortage of human donor pancreases and pancreatic islets, as well as the significant risk associated with transplantation and life-long immunosuppression, the rational differentiation of stem cells into functional beta-cells remains an attractive alternative (Ricordi C, Strom TB. Nat Rev Immunol 4, 259-268 (2004)). Any beta-cell mimetic should be able to store large amounts of insulin and secrete it on demand, as in response to glucose stimulation (Aguayo-Mazzucato C, Bonner-Weir S. Nat Rev Endocrinol 6, 139-148 (2010)). The most effective protocols for the in vitro generation of bonafide pancreatic insulin-secreting beta-like cells that are suitable for transplantation have been the result of sophisticated trial-and-error studies elaborating timely addition of complex growth factor and small-molecule compound cocktails to human pluripotent stem cells (Pagliuca FW, et al. Cell 159, 428-439 (2014); Rezania A, et al., Nature biotechnol 32, 1121-1133 (2014)). Various empirically developed protocols expose all cells to the same stimulatory or blocking components added to the medium but lack the ability to specifically guide and control cell fate decision. The first in vitro differentiation of human pluripotent stem cells into an endodermal, pancreatic lineage was accomplished with identification of critical components like Activin-A, a member of the TGF-beta superfamily or Cyclopamine that are now used in all differentiation protocols (Kroon E, et al. Nature biotechnol 26, 443-452 (2008)).

The most critical and highly variable part of the differentiation procedure is the path from human pancreatic progenitor cells to insulin secreting cells (Rostovskaya M, et al., Philosophical transactions of the Royal Society of London Series B, Biological sciences 370, (2015)). This is reflected by the variety of protocols applied and the inconsistent success rate regarding the generation of insulin secreting pancreatic beta-like cells (Pagliuca FW, et al. Cell 159, 428-439 (2014). ; Rezania A, et al. Nature biotechnology 32, 1121-1133 (2014); Russ HA, et al. EMBO J 34, 1759-1772 (2015)). One of the reasons for these observations could be the heterogeneity in endocrine differentiation and maturation towards a beta-cell phenotype.

Heng BC, et al., Metab Eng, 18, 9-24 (2013) produced mRNA-programmed hIPSCs from adipose tissue-derived mesenchymal cells of a 50 year old donor, demonstrating that the reprogramming (i.e., differentiating) of cells from a donor of advanced age is in principle possible. Recent studies applying similar hESC-based differentiation protocols to human induced pluripotent stem cells (hIPSCs) have produced cells that release human insulin in response to 20 mM glucose. Although the emergence of glucose-sensitive cells was random rather than programmed, this observation suggests that functional pancreatic beta-like cells can eventually be derived from hIPSCs (Pagliuca FW, et al. Cell 159, 428-439 (2014); Rezania A, et al., Nature biotechnol 32, 1121-1133 (2014); Maehr R, et al. PNAS U S A 106, 15768-15773 (2009)).

Ausländer S. et al., Trends in Biotechnology, 31, 155-168 (2013) a review article that relates to the engineering of synthetic networks in living cells describes various ligand-programmable gene switches.

### SUMMARY OF THE INVENTION

The invention provides an expression system useful for establishing a transgenic differentiation-control network as well as methods for differentiating cells. In contrast to the use of growth factor cocktails, differentiation control networks have the advantage of (i) being more economical due to in situ production of the transcription factors required for differentiation, (ii) enabling simultaneous control of ectopic and chromosomally encoded transcription factor variants, (iii) tapping into endogenous pathways and not be limited to cell-surface input, (iv) display improved reversibility that is not dependent on the removal of exogenous growth factors via culture media replacement, (v) providing lateral inhibition of the neighbouring cells, thereby reducing their random differentiation and (vi) enabling trigger programmable and (vii) precising differential transcription factor expression switches.

Such expression system and methods are e.g. useful for differentiating hIPSC-derived pancreatic progenitor cells into glucose-sensitive insulin-secreting beta-cells.

Thus, in a first aspect, the invention relates to an expression system comprising a first receptor molecule for a first ligand, wherein binding of the first ligand to said first receptor molecule controls expression of one or more first expression cassettes via activation of an intracellular signaling cascade, wherein said one or more first expression cassettes encode said second receptor molecule and binding of the first ligand to the first receptor molecule thereby activates expression of the second receptor molecule; and
a second receptor molecule for a second ligand, wherein said second receptor molecule is a transcription factor, wherein binding of the second ligand to said second receptor molecule represses expression of one or more second expression cassettes;
wherein said first and said second ligand are the same; and wherein said first and second receptor molecules have opposing responsiveness to said ligand.

In a second aspect, the invention relates to a transgenic differentiation-control network comprising the expression system described herein.

In a third aspect, the invention relates to an isolated nucleic acid encoding the expression system disclosed herein or a plurality of nucleic acids which in their entirety encode said expression system.

In a forth aspect, the invention relates to a host cell comprising the expression system disclosed herein is provided.

In a fifth aspect, the invention relates to an in vitro method of differentiating a cell, comprising the steps of
(i) Introducing the expression system disclosed herein into said cell;
(ii) Subsequently adding varying doses of one or more ligand(s) to said cell, thereby triggering the sequential expression of two or more genes of interest and altering the phenotype of said cell.

In a sixth aspect, the invention relates to an in vitro method for differentiating a mammalian cell into a pancreatic beta-like cell, comprising the steps of
a) Introducing a set of genetic elements into said mammalian cell, the set of genetic elements forming a differentiation-control network which allows for an inducible expression pattern of the transcription factors Pdx1, Ngn3 and MafA or functional fragments thereof;
b) Exposing the mammalian cell to conditions which allow for the differentially timed expression of said transcription factors, thereby differentiating the mammalian cell into a pancreatic beta-like cell.

Described herein is a cell generated by the methods herein.

Described herein is a cell mixture, an organoid or an artificial organ comprising said cells.

Described herein is a microcontainer comprising said cells.

Described herein is the expression system of the present invention, the nucleic acid(s) disclosed herein, the cell disclosed herein, the cell mixture, artificial organ or microcontainer disclosed herein for use in the treatment of diabetes.

Described herein is a kit comprising the nucleic acid(s) disclosed herein, a mammalian cell line and instructions for use.

### BRIEF DESCRIPTION OF THE DRAWINGS

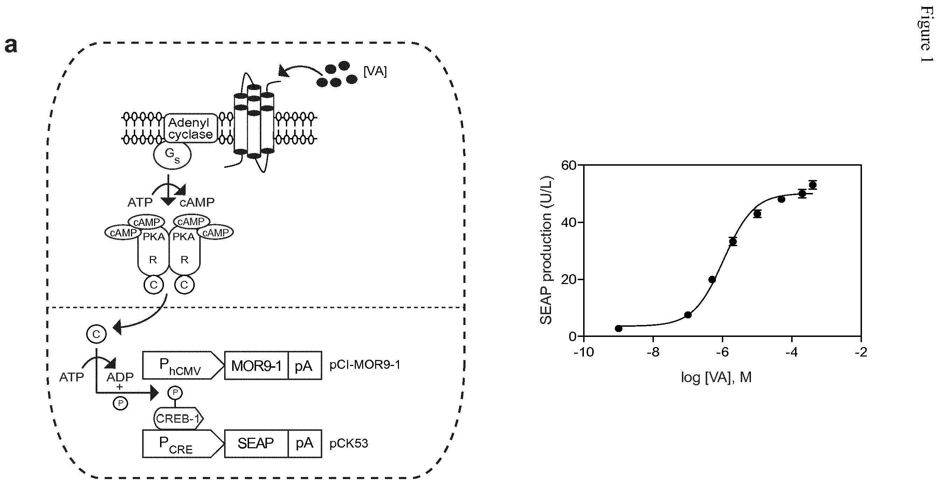

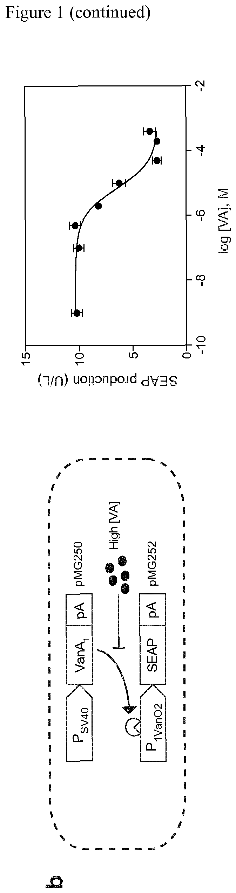

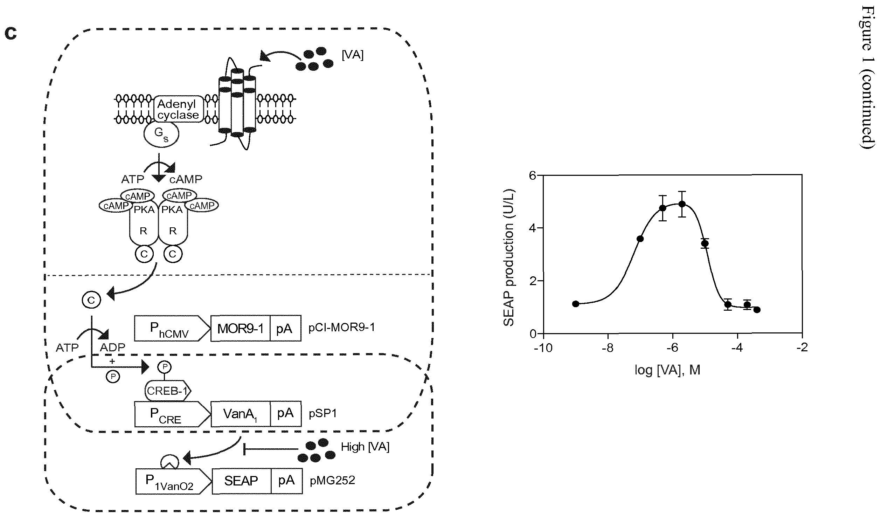

Figure 1 depicts the network components and design of a vanillic acid-responsive positive band-pass filter providing an OFF-ON-OFF expression profile programmed via increasing vanillic acid levels. Figure 1a schematically shows an exemplary first building block of the expression system for vanillic acid-inducible transgene expression. The constitutively expressed vanillic acid sensitive olfactory G protein-coupled receptor MOR9-1 senses extracellular vanillic acids levels and triggers, via a specific G protein (Gs), a vanillic acid-adjusted activation of the membrane-bound adenylyl cyclase that converts ATP into cAMP. When the resulting intracellular cAMP surge produces cAMP-mediated activation of protein kinase (PKA) regulatory subunits, its catalytic subunits are released and translocate into the nucleus where they phosphorylate and actuate cAMP response element-binding protein 1 (CREB1). The activated CREB1 binds to synthetic promoters (P_{CRE}) containing cAMP response elements (CRE) and induces P_{CRE}-driven expression of the reporter gene encoding human placental secreted alkaline phosphatase (SEAP) in response to increasing vanillic acid concentrations. The cotransfection of both, pCI-MOR9-1 and pCK53, in the presence of increasing vanillic acid concentrations results in a dose-inducible SEAP expression profile. Figure 1b shows an exemplary second building block of the expression system for vanillic acid repressible transgene expression. Figure 1c: shows an exemplary positive band-pass expression filter comprising the serial interconnection of the synthetic vanillic acid-inducible signaling cascade of Figure 1a with the vanillic acid-repressible transcription factor-based gene switch of Figure 1b.

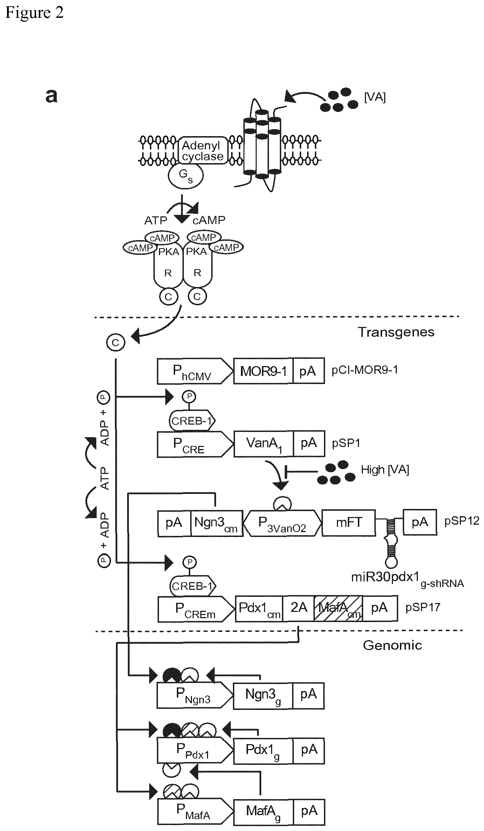

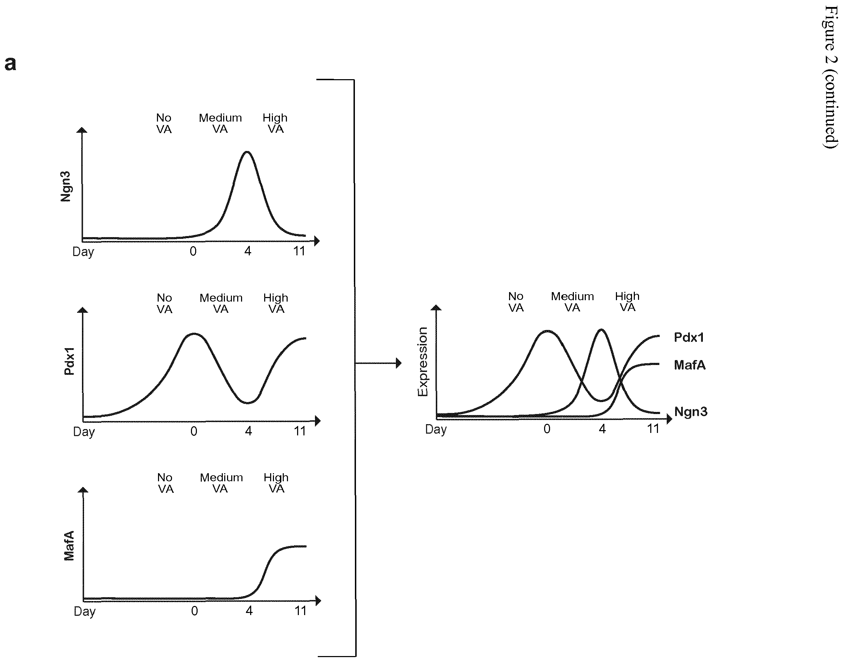

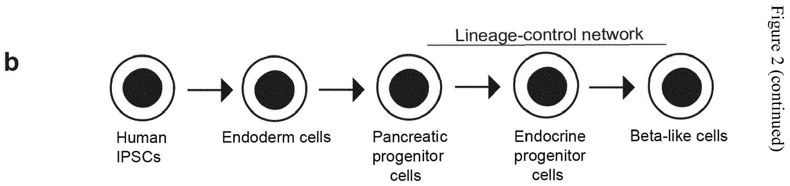

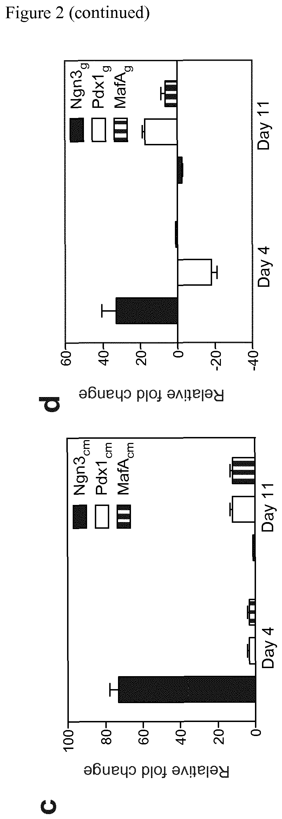

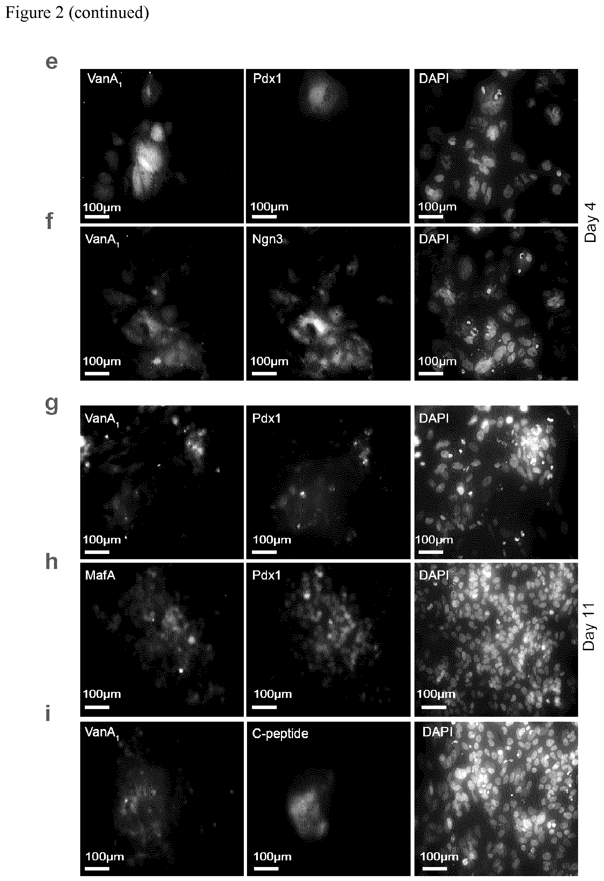

Figure 2 shows the differentiation control network of examples 1 and 2 programming differential expression dynamics of pancreatic transcription factors. Figure 2a schematic depicts the differentiation control network of examples 1 and 2. Figure 2b illustrates the steps involved in differentiating human IPSCs towards pancreatic beta-like cells. Figure 2c show the results of expression profiling of the codon-modified pancreatic transcription factors Ngn3_{cm}, Pdx1_{cm} and MafA_{cm} encoded by the differentiation control network via RT-PCR at days 4 and 11. Data are the means ± SD; n=3. Figure 2d show the results of expression profiling of the genomic pancreatic transcription factors Ngn3_{g}, Pdx1_{g} and MafA_{g} via RT-PCR at days 4 and 11. Data are the means ± SD; n=3. Figures 2e, 2f, 2g and 2h shows immunocytochemistry of pancreatic transcription factors in hIPSC derived pancreatic progenitor cells containing the differentiation control network at days 4 and 11. The cells staining positive for VanA1 were transgenic. Figure 2e: immunocytochemical staining of VanA₁ and Pdxl at day 4. Figure 2f: immunocytochemical staining of VanA1 and Ngn3 at day 4. Figure 2g: immunocytochemical staining of VanA1 and Pdx1 at day 11. Figure 2h: : immunocytochemical staining of MafA and Pdxl at day 11 Figure 2i: : immunocytochemical staining of VanA1, C-peptide and DAPI at day 11.

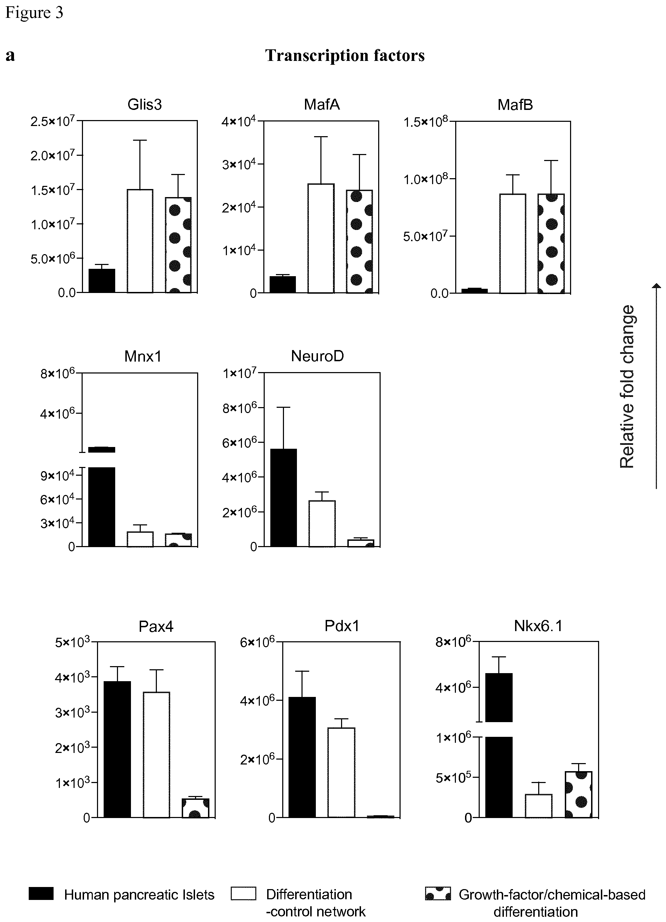

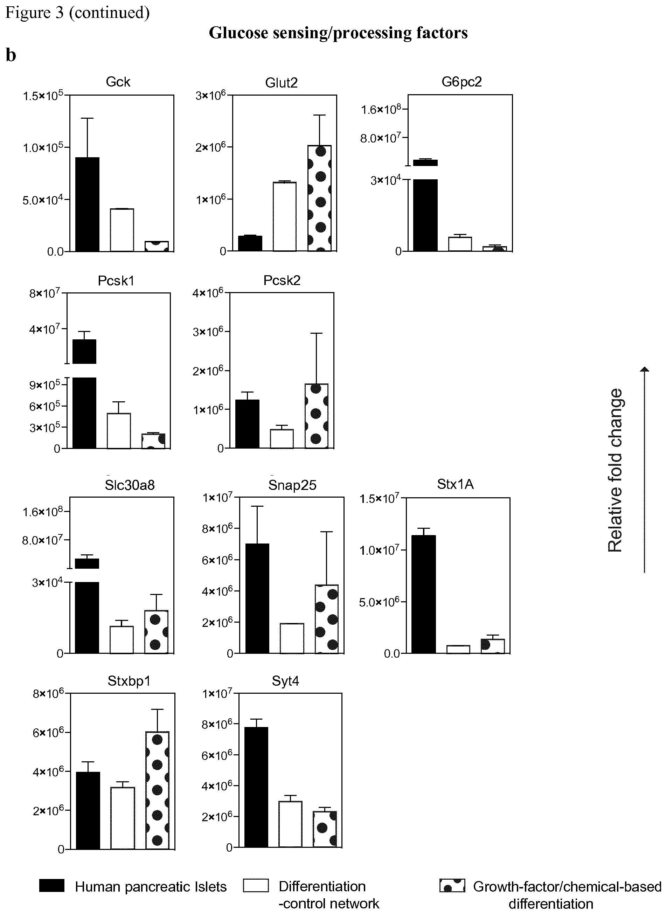

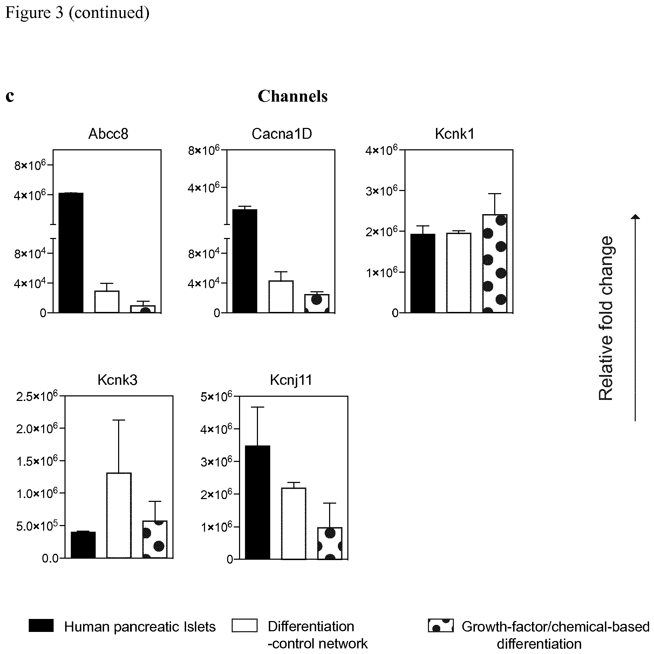

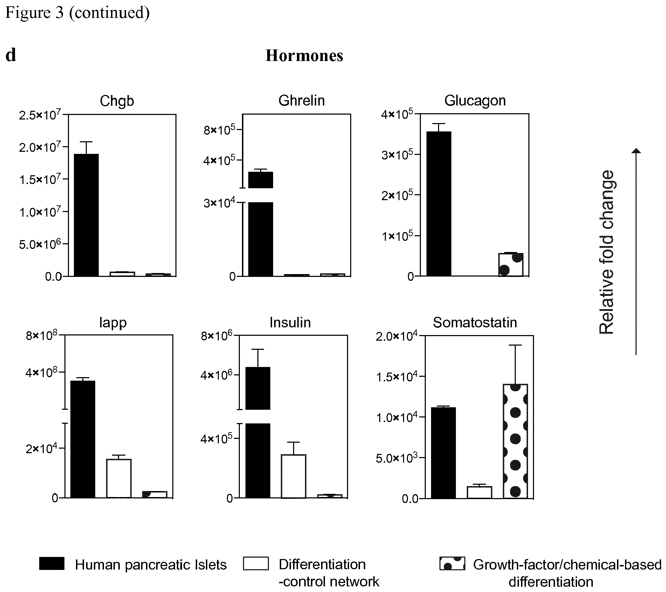

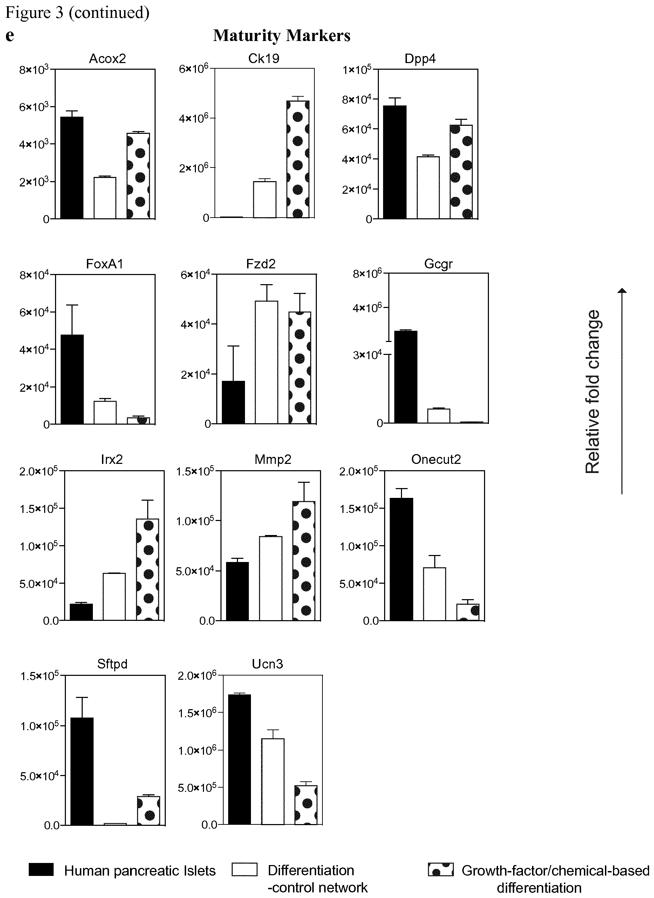

Figure 3 shows qRT-PCR-based gene expression profiling of pancreatic insulin-secreting beta-like cells programmed by the differentiation control network of examples 1 to 3. The transcript levels were profiled at day 11 relative to human IPSCs and normalized to glyceraldehyde 3-phosphate dehydrogenase (GAPDH). Data are the means ± SD; n=3. Figure 3a: expression profiling of key beta-cell-specific transcription factors Glis3, MafA/B, Mnx1, NeuroD, Pax4, Pdxl and Nkx6.1. Figure 3b: expression profiling of glucose- and insulin-processing factors Gck, Glut2, G6pc2, Pcsk1, Pcsk2, S1c30a8, Snap25, Stx1A, Stxbp1, Syt4. Figure 3d: expression profiling of channels Abcc8, Cacna1D, Kcnk1/3 and Kcnj11. Figure 3d: expression profiling of peptide hormones Chgb, Ghrelin, Glucagon, Iapp, Insulin and Somatostatin. Figure 3e: expression profiling of Acox2, Ck19, Dpp4, FoxA1, Fzd2, Gcgr, Irx2, Mmp2, Onecut2, Sftpd and Ucn3.

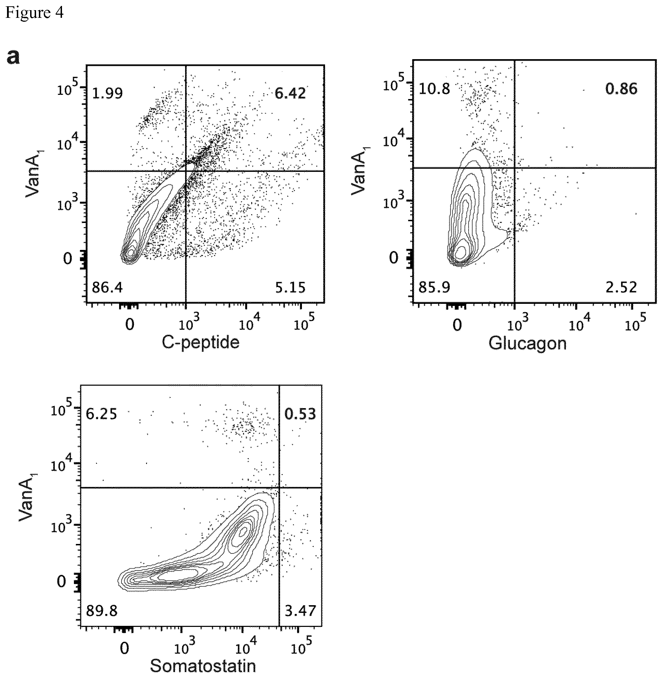

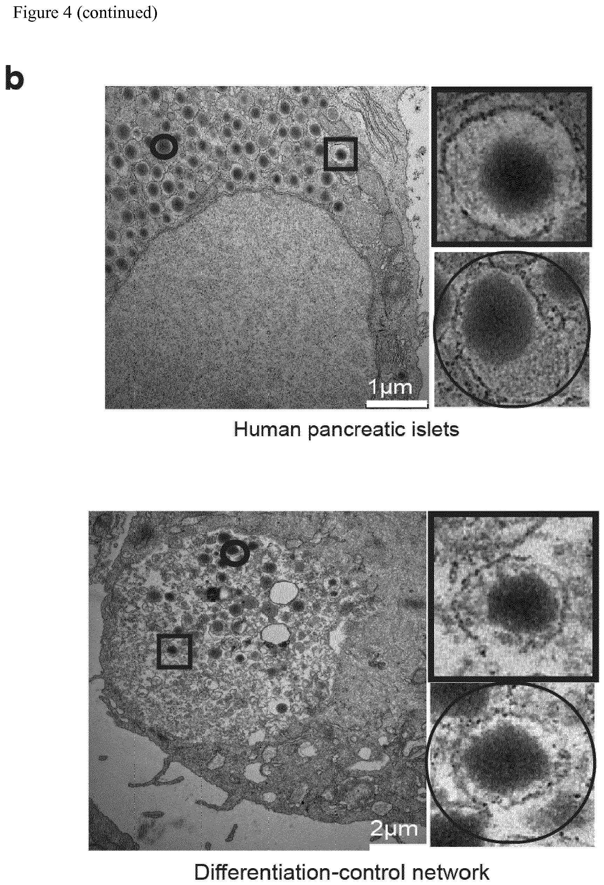

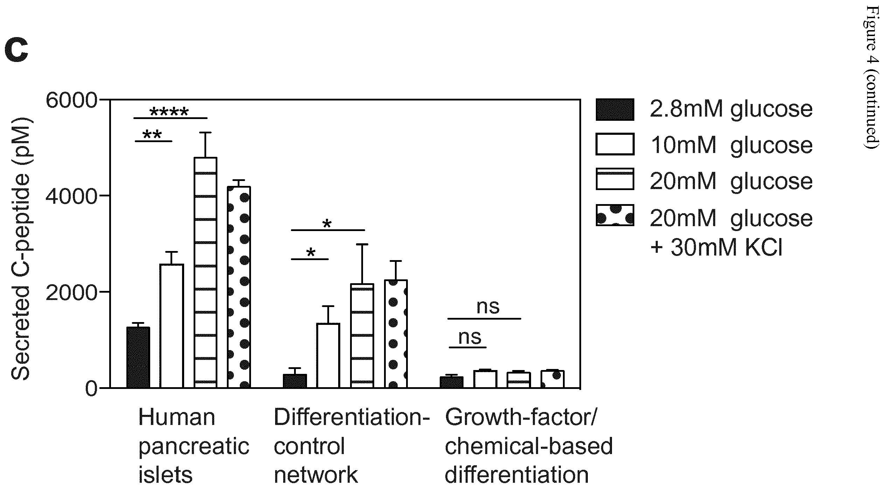

Figure 4 shows the characterization of pancreatic beta-like cells programmed by the differentiation control network. Figure 4a: Representative flow cytometric analysis for VanA₁ with C-peptide (left), Glucagon (middle) and somatostatin (right) for pancreatic beta-like cells on day 11. The cells stained for VanA₁ were transgenic. Figure 4b: Transmission electron microscopy of human islet (left) and differentiation-control network-derived pancreatic beta-like cells (right). Figure 4c: The differentiation-control network-derived pancreatic beta-like cells, control cells and islet were incubated with low glucose (2.8 mM), medium glucose (10 mM), high glucose (20 mM) and high glucose (20 mM) with potassium chloride (30 mM) for 30 min before secreted C-peptide levels were profiled using ELISA and normalized to the total intracellular protein content. Data are the means ± SD; n=3.

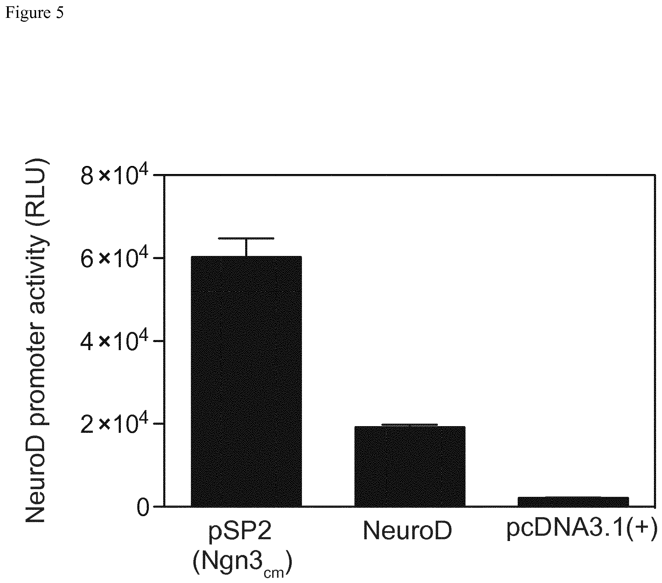

Figure 5 shows the activation of the human NeuroD promoter by Ngn3_{cm} and NeuroD. 2x10⁵ hMSC-TERT were cotransfected with the firefly luciferase (FLuc) reporter construct pPE1FLuc (PE1-FLuc pA) and either the Ngn3cm-encoding pSP2, the NeuroD containing pCMV-NeuroD or pcDNA3.1(+) as negative control and grown for 48h before intracellular luciferase was quantified. Data are mean ± S.D., n=3.

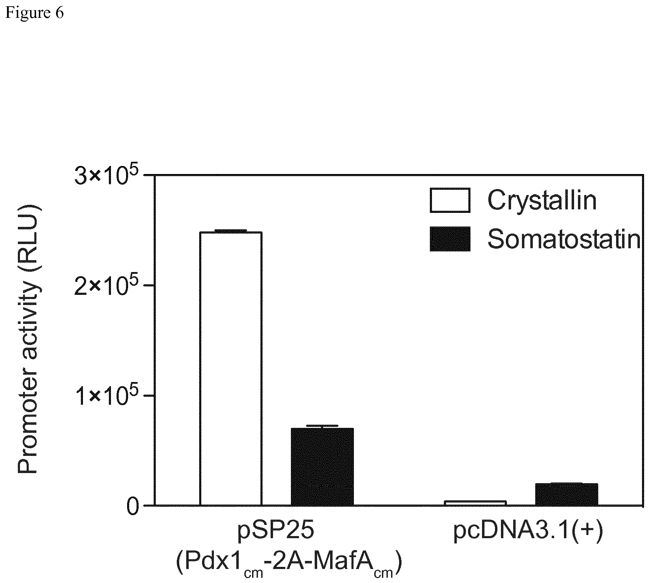

Figure 6 shows the activation of human somatostatin and crystallin promoters by Pdx1_{cm} and MafA_{cm}. 2x10⁵ hMSC-TERT were cotransfected with the constitutive dicistronic Pdx1_{cm} and MafA_{cm} expression vector pSP25 or pcDNA3.1(+) as negative control and either of the somatostatin reporter construct pPSMS900-FLuc or the crystallin reporter vector pPcaA-FLuc and grown for 48h before intracellular luciferase was quantified. Data are mean ± S.D., n=3.

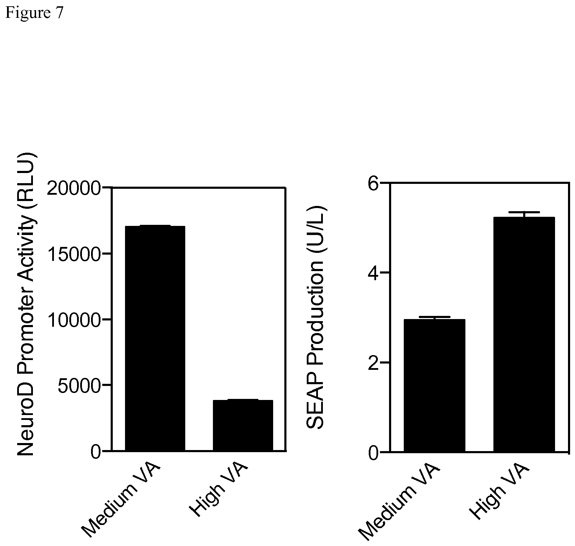

Figure 7 shows the expression levels of Ngn3_{cm} and miR30Pdx1_{g-shRNA} in the presence of medium and high vanillic acid concentrations. 2x10⁵ hMSC-TERT were cotransfected differentiation-control vectors pCI-MOR9-1, pSP1 and pSP12 as well as either the NeuroD promoter-driven firefly luciferase (FLuc)-encoding reporter (PE1-FLuc) or the miR30Pdx1_{g-shRNA}-sensitive SEAP reporter construct pSP14 and grown for 48h in the presence of low (2µM) or high (400µM) vanillic acid (VA) concentrations before intracellular luciferase and secreted SEAP was profiled. Data are mean ± S.D., n=3.

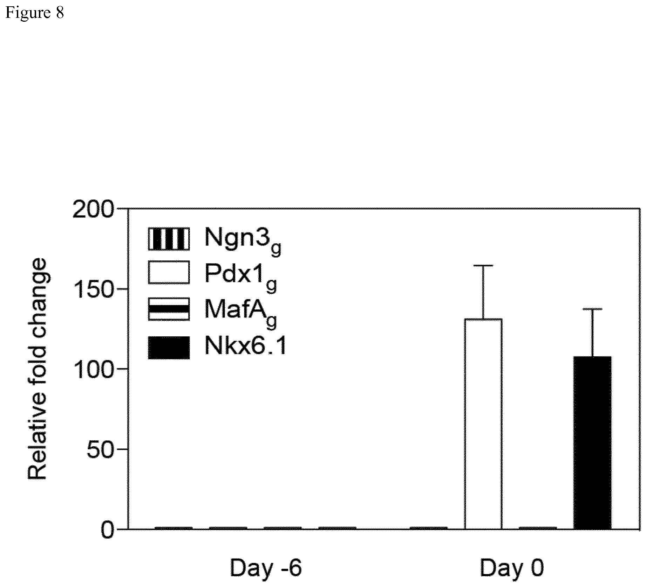

Figure 8 shows qRT-PCR-based expression profiling of the pancreatic transcription factors Ngn3_{g}, Pdx1_{g} and MafA_{g} and Nkx6.1 on days -6 and 0. During the differentiation of hIPSCs to pancreatic progenitor cells the expression of the transcription factors by qRT-PCR relative to day -10 and normalized to GAPDH expression. Data are mean ±S.D.; n=3.

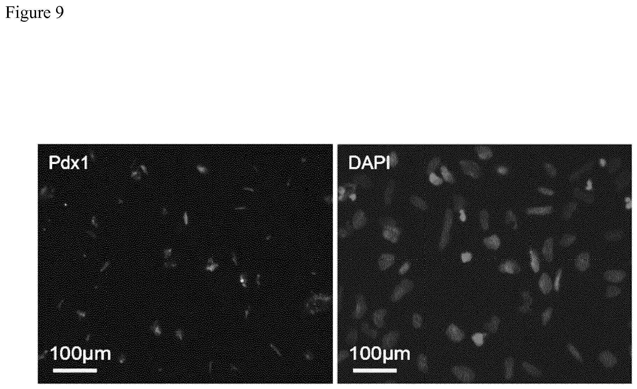

Figure 9 shows Pdx1-specific immunocytochemistry. Pdxl expression of human IPSC-derived pancreatic progenitor cells was confirmed by immunocytochemistry (day 0). DAPI was used to stain the cell nucleus.

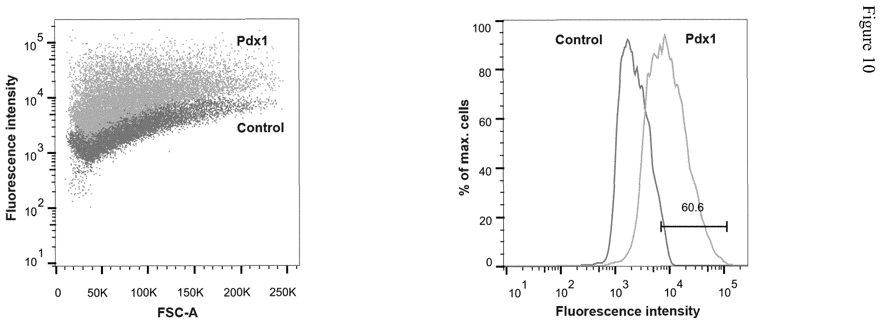

Figure 10 shows FACS-based Pdx1 expression analysis of human IPSC-derived pancreatic progenitor cells at day 0. Undifferentiated human IPSCs were used as control.

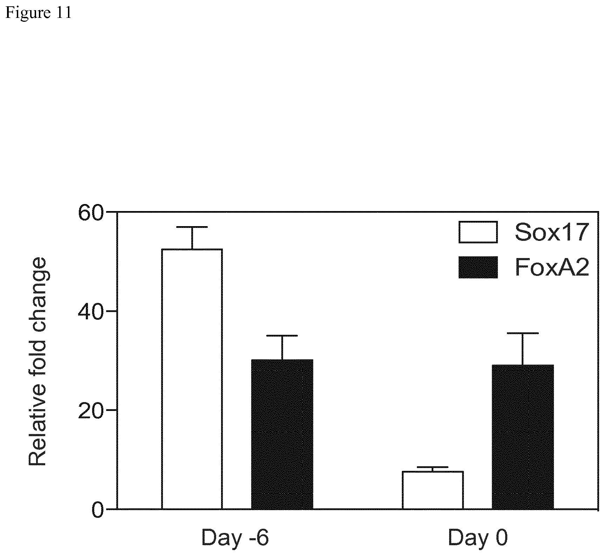

Figure 11 shows qRT-PCR-based expression profiling of Sox17 and FoxA2 on day -6. During the differentiation of hIPSCs to pancreatic progenitor cells the expression of Sox17 and FoxA2 was profiled at day -6 by qRT-PCR relative to day -10 (hIPSC) and normalized to GAPDH expression. Data are mean ±S.D.; n=3.

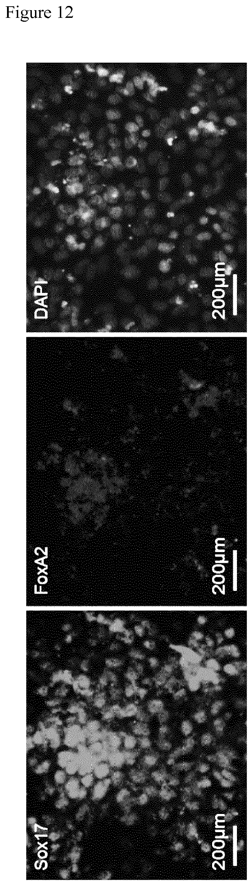

Figure 12 shows Sox17 and FoxA2-specific immunocytochemistry using. During the differentiation of human IPSCs to pancreatic progenitor cells the expression of Sox17 and FoxA2 was profiled at day -6. DAPI was used to stain the cell nucleus.

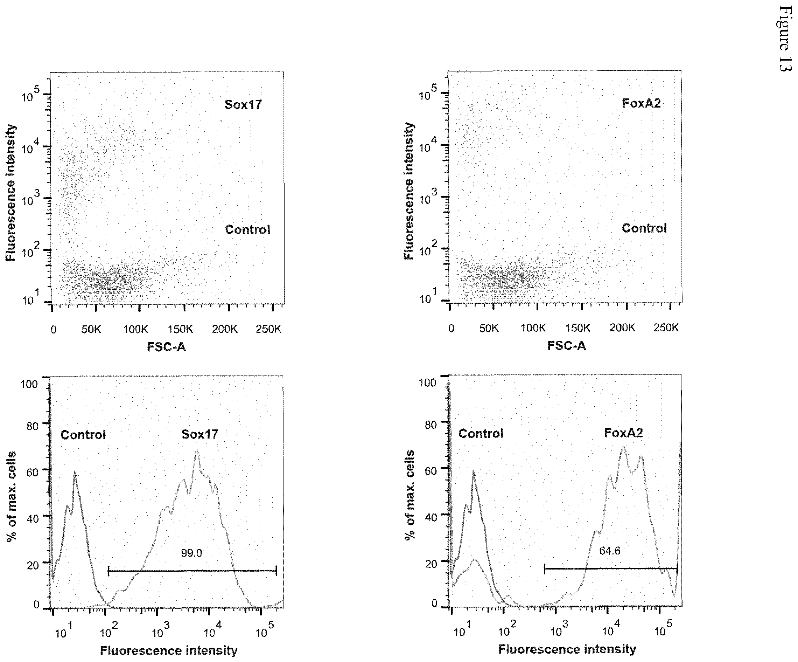

Figure 13 shows FACS-based Sox17 and FoxA2 expression analysis. During the differentiation of human IPSCs to pancreatic progenitor cells the expression of Sox17 and FoxA2 was profiled by FACS analysis at day -6. Undifferentiated human IPSCs were used as control.

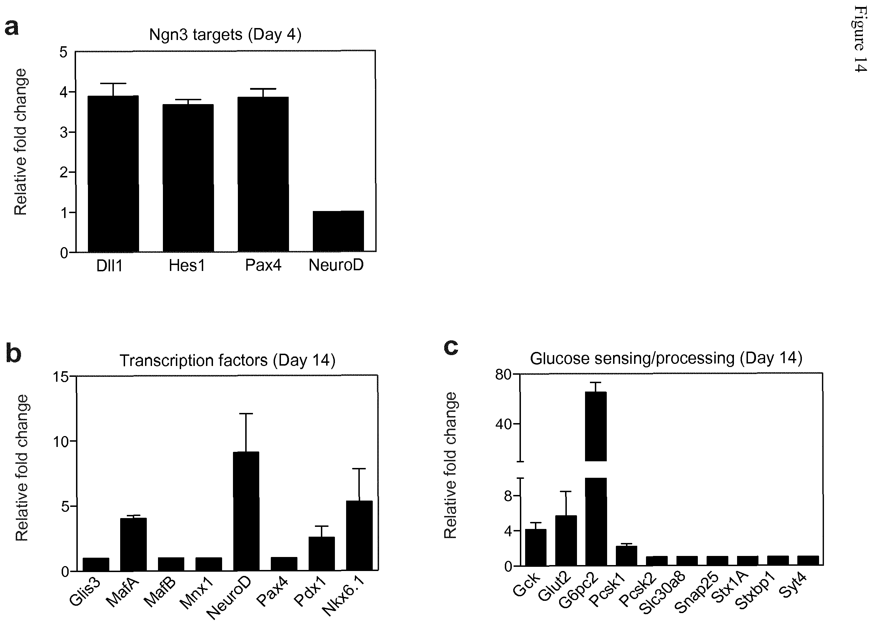

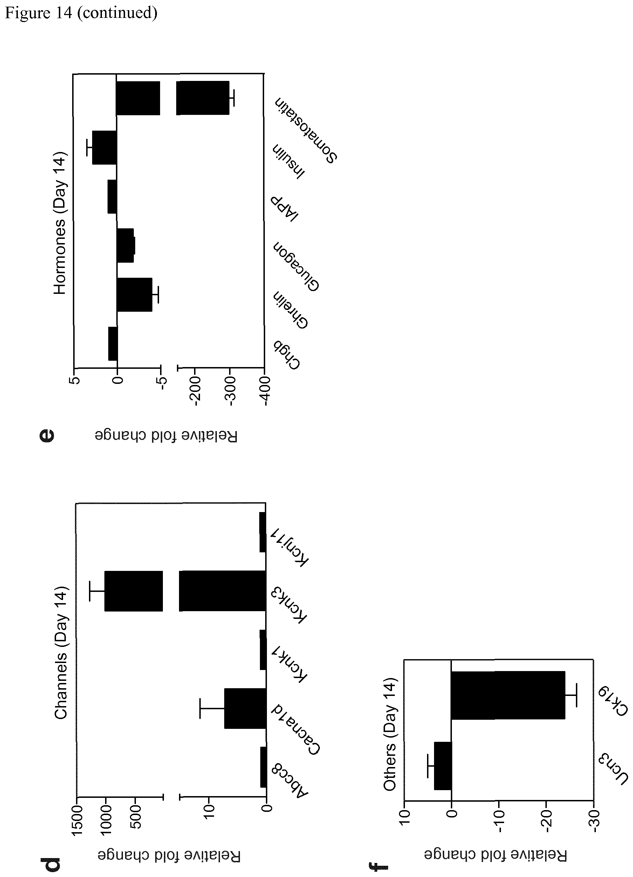

Figure 14 shows differentiation-control network programming expression of pancreatic transcription factors. Figure 14a: RT-PCR-based expression profiling of the Ngn3 target genes Dll1, Hes1, Pax4 and NeuroD 4 days after the kick-off of the differentiation-control network.
Figure 14b: RT-PCR based expression profiling of key beta-cell-specific transcription factors Glis3, MafA/B, Mnx1, NeuroD, Pax4, Pdxl and Nkx6.1. Figure 14c: RT-PCR-based expression profiling of glucose and insulin processing factors Gck, Glut2, G6pc2, Pcsk1, Pcsk2, Slc30a8, Snap25, Stx1A, Stxbp1, Syt4. Figure 14d: RT-PCR based expression profiling of insulin secretion factors Abcc8, Cacna1D, Kcnk1/3 and Kcnj11. Figure 14e: Hormones Chgb, Ghrelin, Glucagon, Iapp, Insulin and Somatostatin. Figure 14f: RT-PCR-based expression profiling of mature beta-cell marker Ucn3 and immature β-cell marker Ck19. The transcript levels were profiled at day 14 relative to randomly differentiating cells and normalized to GAPDH. Data are the means ± SD; n=3.

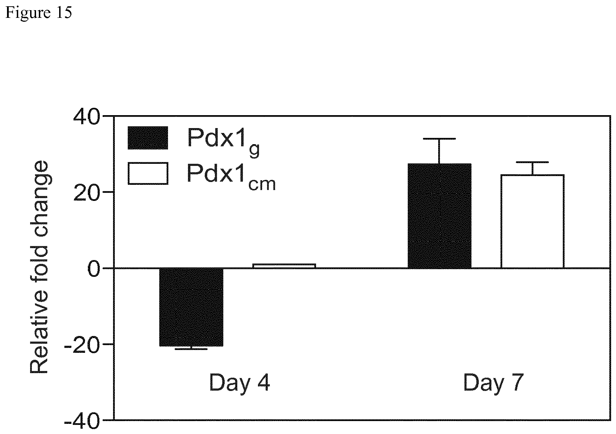

Figure 15 shows recovery of Pdxl levels after switching from miR30Pdx1_{g-shRNA} to Pdx1_{cm} expression. Human pancreatic progenitor cells cotransfected with the differentiation-control network vectors pCI-MOR9-1, pSP1, pSP12 and pSP17 were profiled by qRT-PCR for expression of genomic (Pdx1_{g}) and codon-modified (Pdx-1_{cm}) Pdx1 on days 4 and 7 relative to randomly differentiating cells and normalized to GAPDH expression. Data are mean ±S.D.; n=3.

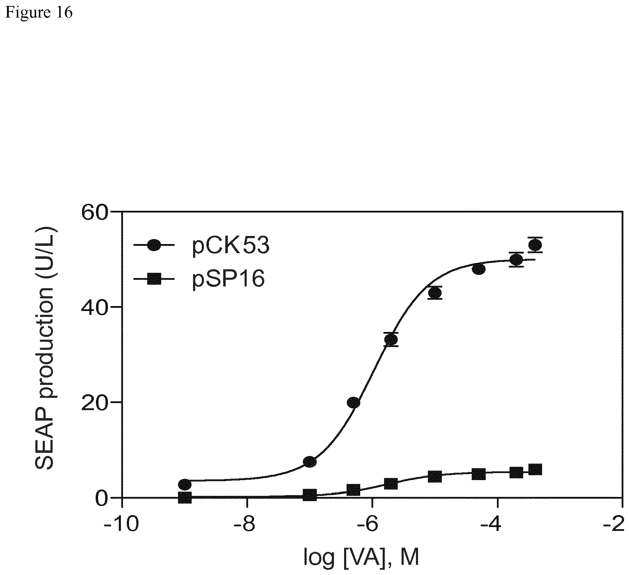

Figure 16 shows the induction kinetics of P_{CRE} and P_{CREm} promoters. 2x10⁵ hMSC-TERT were cotransfected with the constitutive MOR9-1 expression vector pCI-MOR9-1 and either of the P_{CRE} or P_{CREm}-driven SEAP expression vectors pCK53 (P_{CRE}-SEAP-pA) or pSP16 (P_{CREm}-SEAP-pA) and grown for 48h in the presence of different vanillic acid concentrations before SEAP levels were profiled in the culture supernatant. For comparison, the data using pCK53 were replicated from Fig. 1a. Data are mean ± S.D., n=3.

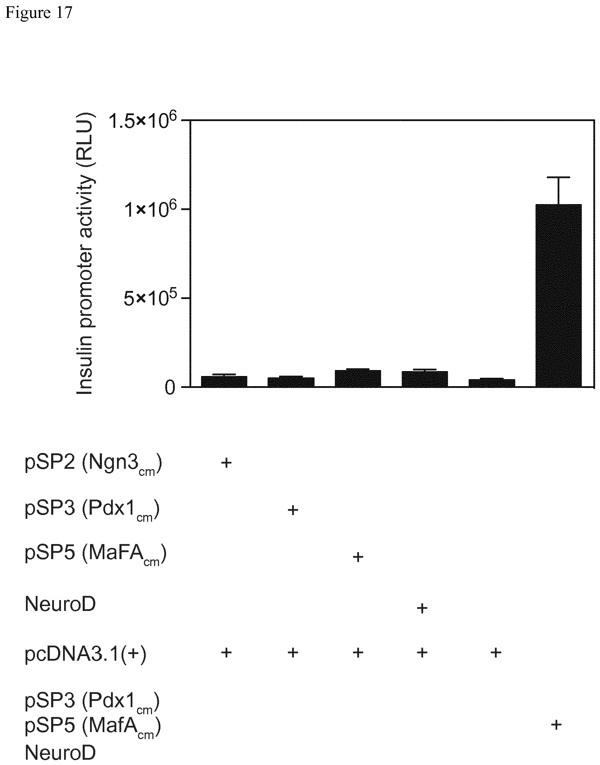

Figure 17 shows the impact of Ngn3, NeuroD, Pdxl and MafA expression on the activity of the human insulin promoter. 2x10⁵ hMSC-TERT were cotransfected with vectors encoding Ngn3 (pSP2), Pdx1 (pSP3), MafA (pSP5), NeuroD (PhCMV-NeuroD - pA), pcDNA3.1(+) and human insulin promoter-driven Gaussia luciferase (GLuc; pSP21) and grown for 48h before GLuc was profiled in the culture supernatant. Data are mean ± S.D., n=3.

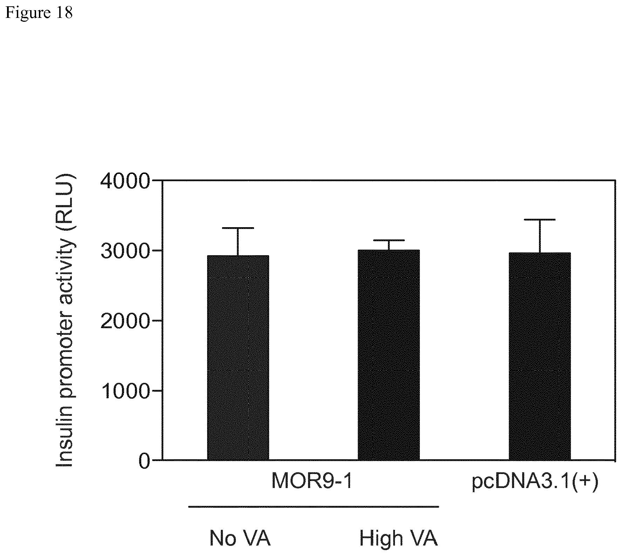

Figure 18 shows the impact of MOR9-1 expression and signaling on the activity of the human insulin promoter. 2x10⁵ hMSC-TERT were cotransfected with pCI-MOR9-1 or pcDNA3.1(+) and pSP21 and grown for 48h in presence (+; 400µM) and absence of (-) vanillic acid before Gaussia luciferase (GLuc) was profiled in the culture supernatant. Data are mean ± S.D., n=3.

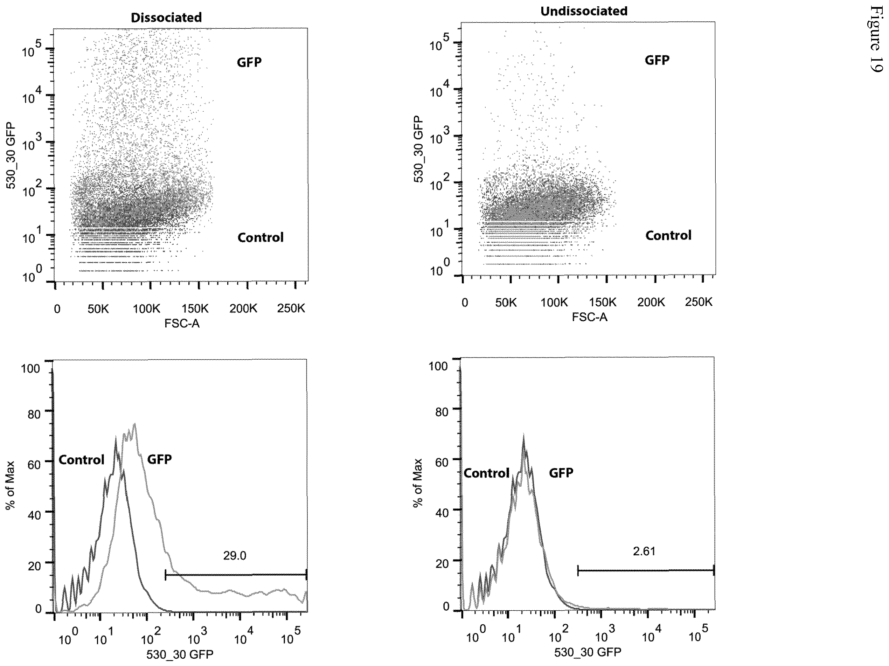

Figure 19 shows the FACS-based analysis of the transfection efficiency of human pancreatic progenitor cells. Comparative flow-cytometric analysis of dissociated and non-dissociated native and pEGFP-N1-transfected human IPSC-derived pancreatic progenitor cells. Non-transfected human pancreatic progenitor cells were used as control.

### DETAILED DESCRIPTION

So that the invention may be more readily understood, certain terms are first defined. Unless otherwise defined within the specification, all technical and scientific terms used herein have their art-recognized meaning. Although similar or equivalent methods and materials to those described herein can be used in the practice or testing of the invention, suitable methods and materials are described below. In case of conflict, the present specification, including definitions, will prevail. The materials, methods, and examples are illustrative only and not intended to be limiting.

As used herein, the term "pancreatic beta-like cell", "beta-like cell", "pancreatic β-like cell" or "pancreatic β-like cell" refers to a cell which is artificially reprogramed or differentiated, e.g. by the methods as disclosed herein, to expresses markers of mature endogenous pancreatic beta-cells. In particular, such pancreatic beta-like cell is capable of expressing insulin.

As used herein, the term "beta-cell" refers to a cell type found in the pancreas, in particular in the human pancreatic islets. Beta cells are the primary producers of insulin. A beta cell inter alia expresses the marker molecules Nkx6.1, MafA, Ucn3, G6pc2, c-peptide, glucose transporter 2 (Glut2), glucokinase (GCK), prohormone convertase 1/3 (PC1/3), β-cell transcription factors NeuroD and Nkx2.2.

As used herein, the term "MOR9-1" refers to an olfactory receptor, preferably an olfactory receptor as encoded by SEQ ID No. 32. Also known as Olfr609, said receptor is an olfactory receptor derived from mouse (mus muslus; GenBank accession number: AY073004) which is sensitive to vanillic acid. MOR9-1 is G protein-coupled.

As used herein, the term "VanA₁" refers to a synthetic transcription factor, the vanillic acid dependent transactivator, preferably the vanillic acid dependent transactivator as encoded by SEQ ID No. 33. Specifically, it is a fusion protein of the vanillic acid-dependent repressor VanR, preferably of the vanillic acid-dependent repressor VanR as encoded by SEQ ID No. 34, and the transactivation domain of VP16. However, VP16 can be exchanged by any transactivation domain or functional fragments thereof, such as p65, VP64 or E2F4 without being limited thereto. The fusion protein may comprise a linker between the repressor and the transactivation domain.

As used herein, the term "Gs" refers to a specific protein of the G-protein type family (GenBank accession number: X04408). Typically, G proteins trigger a complex network of signaling pathways within cells.

As used herein, the term "CREB1" refers to the transcription factor "cAMP responsive element binding protein 1". The protein is a member of the leucine zipper family of DNA binding proteins which binds as a homodimer to the cAMP-responsive element. CREB1 is phosphorylated by several protein kinases, and induces transcription of genes through binding to the promotor P_{CRE} in response to activation of the cAMP pathway.

As used herein, the term "miR30Pdx1_{g-shRNA}" refers to a small hairpin RNA which exclusively targets genomic Pdxl (Pdx1_{g}) transcripts for RNA interference-based destruction.

As used herein, the term "vanillic acid" refers to the dihydroxybenzoic acid derivative "4-hydroxy-3-methoxybenzoic acid". The compound is an oxidized form of vanillin. Vanillic acid is used as a food additive, in particular as flavoring agent.

As used herein, the term "expression system" refers to a set of transgenic genetic elements within a cell as well as proteins encoded by such genetic elements.

As used herein, the term "differentiation-control network" refers to an engineered composition within a cell that comprises at least one expression system which may or not interact with the cells endogenous regulatory networks. Such synthetic control network can perform several functions including a sensing and a regulatory function.

As used herein, the term "expression cassette" refers to a nucleic acid molecule that is capable of directing transcription. An expression cassette includes, at the least, a promoter or a structure functionally equivalent thereto as well as the coding sequence of a gene of interest. Additional elements, such as an enhancer, a transcription termination signal and/or a polyadenylation signal, may also be included.

As used herein, the term "Pdx1" refers to the transcription factor "pancreatic and duodenal homeobox 1", also known as insulin promoter factor 1, preferably the pancreatic and duodenal homeobox 1 as encoded by SEQ ID No. 29 (see also GenBank accession No: NP-000200.1 for human Pdx1). Pdx1 acts as transcriptional activator of insulin, somatostatin, glucokinase, islet amyloid polypeptide, and glucose transporter type 2 (GLUT2). As such, Pdxl is necessary for early pancreatic development, including β-cell maturation, and duodenal differentiation. The term also encompasses variants, homologues, allelic forms, mutant forms, and equivalents thereof, having, e.g., conservative substitutions, additions, deletions within its sequence which not adversely affecting the structure or function of the molecule.

As used herein, the term "MafA" refers to a transcription factor also known as proto-oncogene c-Maf or V-maf musculoaponeurotic fibrosarcoma oncogene homolog (see GenBank accession number NP-963883.2 for human MafA), preferably a proto-oncogene c-Maf (MafA) as encoded by SEQ ID No. 28. MafA binds RIPE3b, a conserved enhancer element that regulates pancreatic beta cell-specific expression of the insulin gene. MafA is known to activate gene transcription of Glut2, pyruvate carboxylase, Glut2, Pdx-1, Nkx6.1, GLP-1 receptor and prohormone convertase-1/3 (see Wang et al., Diabetologia. 2007 February; 50(2): 348-358). The term also encompasses variants, homologues, allelic forms, mutant forms, and equivalents thereof, having, e.g., conservative substitutions, additions, deletions within its sequence which not adversely affecting the structure or function of the molecule.

As used herein, the term "Ngn3", refers to the neurogenin 3 protein which is expressed in endocrine progenitor cells and is required for endocrine cell development in the pancreas (see GenBank acc No. NP_066279.2 for human Ngn3), preferably the neurogenin 3 protein as encoded by SEQ ID No. 27. It belongs to a family of basic helix-loop-helix transcription factors involved in the determination of neural precursor cells in the neuroectoderm. As a transcription factor, Ngn3 targets Dill, Hes1, Pax4 and NeuroD. Ngn3 is also known as aliases, Atoh5, Math4B, bHLHa7, and NEUROG3. The term also encompasses variants, homologues, allelic forms, mutant forms, and equivalents thereof, having, e.g., conservative substitutions, additions, deletions within its sequence which not adversely affecting the structure or function of the molecule.

As used herein, the term "functional fragments" of Pdx1, Ngn3 and/or MafA refers to proteins having a similar amino acid sequences thereto, and are optionally shorter or longer as the respective wildtype sequence, wherein the functional fragment is about at least 0.7 fold, 0.8 fold, 0.9 fold, 1 fold, 1.2 fold, 1.5-fold or greater than 1.5-fold as effective at differentiating cells as the corresponding wild type Pdx1, Ngn3 or MafA. The functional fragment polypeptide may have additional functions that can include decreased antigenicity and/or increased DNA binding.

As used herein, the term "insulin" refers to the protein hormone produced by beta cells in the pancreas which decreases blood glucose concentrations and is therefore involved in the regulation of blood sugar levels. Insulin is produced as a proinsulin precursor which is , consisting of the B and A chains of insulin linked together via a connecting C-peptide. Insulin itself is comprised of only the B and A chains. Human insulin is encoded by the INS gene corresponding to GenBank Accession No: NM-000207.2.

As used herein, "hMSC-Tert" refers to human mesenchymal stem cells transgenic for the catalytic subunit of human telomerase (Simonsen et al., Nat. Biotechnol. 20, 592-596 (2002)).

As used herein, "Fluc" refers to firefly luciferase.

As used herein, "GAPDH" refers to glyceraldehyde 3-phosphate dehydrogenase.

As used herein,"DAPI" refers to Diamidino-2-phenylindole.

As used herein, the term "exogenous" refers to any material that is present in a cell or an organism which is not native to said cell or organism but originates outside that cell or organism, as opposed to "endogenous".

The term "stem cell" as used herein refers to undifferentiated biological cells that can differentiate into specialized cells and which is capable of proliferation to produce more stem cells.

As used herein, the term "somatic cell" refers to any cell forming the body of an organism, as opposed to germline cells or undifferentiated stem cells.

As used herein, the term "IPSC" refers to induced pluripotent stem cells, also known as "iPS cell" or "induced pluripotent stem cell". It refers to a pluripotent stem cell which is artificially derived from a non-pluripotent cell such as an adult somatic cell. "hIPSC" refers to human induced pluripotent stem cells.

As used herein, the term "endoderm cell" refers to a cell which is from one of the three primary germ cell layers in the very early embryo other than the mesoderm or ectoderm. Endoderm cells ultimately differentiate into the liver and pancreas.

As used herein, the term "pancreatic progenitor cell" refers to a cell of the pancreas, which is already more specific than a stem cell but not yet differentiated into its mature "target" cell. Progenitor cells can divide only a limited number of times.

As used herein, an "endocrine progenitor cell" refers to a multipotent cell of the definitive endoderm lineage that expresses at least a marker from the list consisting of neurogenin 3 (NEUROG3), Pdxl, Ptf1A, Sox9, Nkx6.1, Hnf6, FoxA1, FoxA2, GatA6, Myt1, Islet1, Pax4, Pax6, Nkx2.2, MafA and MafB without being limited to, which can further differentiate into cells of the endocrine system including, but not limited to, beta-cells or pancreatic beta-like cells.

In a first aspect, an expression system comprising a first receptor molecule for a first ligand and a second receptor molecule for a second ligand, wherein said second receptor molecule is a transcription factor, wherein binding of the first ligand to said first receptor molecule controls expression of one or more first expression cassettes via activation of an intracellular signaling cascade, wherein said one or more first expression cassettes encode said second receptor molecule and binding of the first ligand to the first receptor molecule thereby activates expression of the second receptor molecule; and binding of the second ligand to said second receptor molecule represses expression of one or more second expression cassettes; wherein said first and said second ligand are the same; and
wherein said first and second receptor molecules have opposing responsiveness to said ligand. Such expression system provides for an inducible time-delay expression pattern of one or more genes of interest which are endogenous to the cell and/or exogenous to the cell. Such expression system is e.g. useful for differentiating cells.

The first and second receptor molecule may both be expressed on the cell surface. Alternatively, the first receptor molecule is expressed on the cell surface and the second receptor molecule intracellularly.

As used herein, the term "receptor molecule" refers to a protein molecule which is capable of binding a ligand and which upon binding, changes its conformation and functionality. Receptors on the cell surface typically are membrane-spanning proteins whereas intracellulary expressed receptor molecules may be transcription factors or enzymes (such as e.g. isomerases or kinases).

Binding of a ligand to a cell surface receptor molecule, such as binding of the first ligand to the first receptor molecule, activates a signaling cascade. In some embodiments, said signaling cascade triggers activation of a transcription factor. In some embodiments, said transcription factor activates expression of the second receptor molecule. Thereby, the first receptor molecule activates, i.e. controls, expression of the second receptor molecule. Thus in a preferred embodiment the first receptor molecule activates, i.e. controls, expression of the second receptor molecule.

The first and/or the second receptor molecule may be constitutively expressed. Suitable promotors for constitutive expression include the human cytomegalo-virus immediate early promoter P_{hCMV} and/or the simian virus 40 promoter P_{SV40}. In a preferred embodiment, the first receptor molecule is constitutively expressed whereas expression of the second receptor molecule is inducible.

In some embodiments, the first and second receptor molecules are different to each other. In a preferred embodiment, the first and second receptor molecules are different to each other and the first receptor molecule is expressed on the cell surface, e.g. is preferably a membrane-spanning protein and the second receptor molecule is expressed intracellularly e.g. is preferably an intracellular receptor molecule located inside the cell.

The second receptor molecule itself is a transcription factor. Said transcription factor may activate transcription of the one or more second expression cassettes, typically via an inducible promotor.

Binding of the second ligand to the second receptor molecule represses expression of said second one or more expression cassettes.

The first and/or second expression cassettes encode proteins and/or regulatory elements of interest. Exemplary regulatory elements include, without being limited to, transcription factors (such as Pdxl, Ngn3 or MafA, or respective functional fragments thereof), hairpin RNA, micro RNAs, siRNA, aptamers, ribozymes, riboswitches, guide RNAs (CRISPR/Cas9) and antisense RNA. Such regulatory elements may either activate (e.g., the transcription factors) or repress transcription (such as hairpin RNA, antisense RNA etc.) of an endogenous or exogenous gene of interest. For example, regulatory elements encoded by a second expression cassette may interfere with the one or more first expression cassettes controlled by the first receptor molecule. In some embodiments, the first and/or second expression cassettes encode more than one protein and/or regulatory elements. For example, the promotor controlling expression of such expression cassette may be bidirectional and encode two or more regulatory elements selected from the group consisting of transcription factors (such as Pdxl, Ngn3 or MafA, or respective functional fragments thereof), hairpin RNA, micro RNAs, siRNA, aptamers, ribozymes, riboswitches, guide RNAs (CRISP/Cas9) and antisense RNA. Such regulatory elements may serve to control expression of chromosomal genes (i.e. endogenous genes) and/or exogenous genes.

In the expression cassettes, a polyadenylation site (pA), such as the SV40-derived late polyadenylation site, may be present.

As used herein, the term "ligand" refers to any signaling-triggering molecule capable of binding to a receptor with a certain affinity and activates or inhibits receptor activity. A ligand is preferably an exogenous ligand, i.e. it is not produced by the cell comprising the expression system. The ligand may be a small molecule, a peptide or a protein. Preferably, the ligand is nontoxic. Table 1 on pages 157 to 159 of Ausländer and Fussenegger, Trends Biotechnol. 31, pp. 155-168 (2013), enumerates suitable ligands and their corresponding receptors. In one embodiment, the ligand is a food additive, such as vanillic acid. Vanillic acid is a natural plant component and licensed as food additive. Therefore, it is a suitable candidate for biopharmaceutical manufacturing scenarios as well as in gene- and cell-based therapies using the expression system described herein. In another embodiment, the ligand is tetracycline. In still another embodiment, the ligand is erythromycin. In still another embodiment, the ligand is selected from the group consisting of benzoic acid, biotin, parabens, phloretin, pristinamycin, butyrolactone, L-tryptophan, 6-hydroxy-nicotine, light, dopamine, protons, CO₂, L-arginine, uric acid, 2-phenyl-ethyl-butyrate, bile acid, streptogramin, macrolide trimethoprim and vanillic acid. Gene regulation systems and/or their corresponding ligands such as a uric acid-responsive expression system (UREX), a butyrolactone-responsive expression system (Quorex), a L-arginine-responsive expression system (ART), a L-tryptohan-responsive expression system (TRT), a streptogramin-responsive expression system (PIP), a tetracycline-responsive expression system (TET), a macrolide-responsive expression system (E.REX), a trimethoprim-responsive expression system (TMP DD), a phloretin-responsive expression system (PEACE) and a vanillic acid-response expression system (VAC) are well known in the art, see e.g. Ausländer and Fussenegger, Trends Biotechnol. 31, pp. 155-168 (2013) and Gitzinger M, et al, Nucleic Acids Research, Vol. 40(5) e37 (2012) for further reference. Preferably gene regulation systems and/or their corresponding ligands are selected from the group consisting of a uric acid-responsive expression system (UREX), a butyrolactone-responsive expression system (Quorex), a L-arginine-responsive expression system (ART), a L-tryptohan-responsive expression system (TRT), a streptogramin-responsive expression system (PIP), a tetracycline-responsive expression system (TET), a macrolide-responsive expression system (E.REX), a trimethoprim-responsive expression system (TMP DD), a phloretin-responsive expression system (PEACE) and a vanillic acid-response expression system (VAC), more preferably selected from the group consisting of a tetracycline-responsive expression system (TET), a macrolide-responsive expression system (E.REX), a trimethoprim-responsive expression system (TMP DD), a phloretin-responsive expression system (PEACE) and a vanillic acid-response expression system (VAC), most preferably selected from the group consisting of a phloretin-responsive expression system (PEACE) and a vanillic acid-response expression system (VAC).

The first and the second ligand are the same. In such setting said first and second receptor molecules have opposing responsiveness to said ligand. Additionally or alternatively, said first and second receptor molecules have differential sensitivity to said ligand.

In some embodiments, said first receptor molecule is MOR9-1.

Upon binding of the exogenous first ligand to the first receptor molecule, a signaling cascade is triggered within the cell comprising the expression system, such signaling cascade comprises in some embodiments:
(i) Activation of the Gs alpha subunit to activate cAMP production via adenylyl cyclase;
(ii) Phosphoactivation and binding of cAMP to CREB1; and/or
(iii) Activation of CRE mediated transcription of the one or more expression cassettes under control of the first receptor molecule. In such embodiments, the one or more expression cassettes under control of the first receptor molecule, such as the expression cassette encoding the second receptor molecule, are cAMP responsive.

In some embodiments, the second receptor molecule is vanillic acid-dependent transactivator VanA₁, preferably the vanillic acid-dependent transactivator VanA₁ as encoded by SEQ ID No. 33. In such embodiments, it is preferred that the one or more second expression cassettes being controlled by the second receptor molecule comprises a vanillic acid-responsive promotor.

Gitzinger M, et al, Nucleic Acids Research, Vol. 40(5) e37 (2012) describes one exemplary building block of the expression system, namely the vanillic acid repressible transgene expression building block of Figure 1b. Said building block was compatible with other transgene regulation systems that capitalize on different inducers, i.e. tetracycline- (TETOFF) or the erythromycin- (EOFF) responsive expression systems. Both, the TETOFF and the EOFF-responsive expression systems are based on intracellular receptor molecules.

In some embodiments, the expression system comprises the building block shown in Figure 1b. The constitutively expressed, vanillic acid-dependent transactivator VanA₁, binds and activates the chimeric promoter P_{1VanO2} (encoded by pMG252) in the absence of vanillic acid. In the presence of increasing vanillic acid concentrations, VanA₁ is gradually released from P_{1VanO2}, and transgene expression is shut down in a dose-dependent manner. The cotransfection of both constructs in presence of increasing vanillic acid concentrations results in a dose repressible expression profile of the reporter gene SEAP.

In some embodiments, binding of the first ligand to the first receptor molecule triggers a signaling cascade within the host cell, thereby activating expression of the first expression cassette encoding Pdxl and MafA. Additionally, the second expression cassette encodes for Ngn3. Binding of the second ligand to the second receptor molecule represses Ngn3 expression. In contrast to the current beta-cell differentiation protocols where the various components in the medium (chemicals and growth factors) expose all cells to the same stimuli, the expression system of the present invention allows to precisely execute the differentiation program starting with activation of Ngn3 and followed by repression of Ngn3 and subsequent activation of Pdx1 and MafA in the same cell. In some embodiments, the one or more transcription factors encoded by the first or second expression cassette are codon modified (cm). Codon modification allows for characterization of the relative contributions of the chromosomal and heterologous Pdx1, MafA and/or Ngn3 to the differentiation.

In one embodiment, the expression system comprises an expression cassette encoding MOR9-1 as first receptor under the control of a constitutive promotor. Further, an expression cassette encoding the vanillic acid dependent transactivator VanA1 under control of a cAMP-responsive expression cassette is provided. Moreover, an expression cassette under control of VanA1 is provided which encodes at least Ngn3 or a functional fragment thereof as well as a regulatory element capable of repressing endogenous Pdx1 expression. The expression system further comprises a cAMP-responsive expression cassette encoding Pdxl and/or MafA or functional fragments thereof, respectively.

A particular, exemplary embodiment of the expression system of the invention is shown in Figure 1c. Herein, both building blocks as described above are serially combined, i.e. the synthetic vanillic acid-inducible signaling cascade of Figure 1a with the vanillic acid-repressible transcription factor-based gene switch of Figure 1b. Both receptors bind and respond to the same ligand, vanillic acid. Due to the opposing responsiveness and differential sensitivity to vanillic acid, this synthetic gene network programs a positive band-pass filter expression of the marker gene SEAP profile (OFF-ON-OFF) as vanillic acid levels are gradually increased. Medium levels of the ligand vanillic acid, i.e. 10 nM to 2 µM) activate the first receptor MOR9-1, which induces P_{CRE}-driven VanA₁ expression. VanA₁ acts as second receptor molecule and has a different and opposing sensitivity towards vanillic acid. At medium concentrations of vanillic acid, VanA₁ remains active and acts in a feed forward manner to trigger P_{1VanO2}-mediated expression of the expression cassette encoding the reporter gene SEAP, which gradually increases to maximum levels. At high vanillic acid concentrations, i.e. above 2 µM, MOR9-1 maintains P_{CRE}-driven VanA₁ expression, but VanA₁ dissociates from P_{1VanO2}, which results in a gradual shutdown of SEAP expression. The cotransfection of all three expression plasmids in presence of increasing vanillic acid concentrations programs the engineered cells to produce a positive band-pass filter expression profile of the reporter gene SEAP (OFF-ON-OFF).

Another exemplary embodiment of the expression system is described in Figure 2a. MOR9-1 acting as first receptor molecule is constitutively expressed from the expression vector pCI-MOR9-1. When the exogenous ligand vanillic acid, a synthetic signaling cascade is triggered, inducing P_{CRE}-driven expression of the transcription factor VanA₁, the second receptor molecule, expressed from pSP1. At medium vanillic acid concentrations (between 10 nM and 2 µM), VanA₁ binds and activates the bidirectional vanillic acid-responsive promoter P_{3VanO2} on pSP12, which drives the induction of codon modified Neurogenin 3 (Ngn3_{cm}) as well as the coexpression of both the blue-to-red medium fluorescent timer (mFT) for precise visualization of the unit's expression dynamics and a small hairpin RNA programming the exclusive destruction of genomic pancreatic and duodenal homeobox 1 (Pdx1_{g}) transcripts (miR30pdx1_{g-shRNA}). Consequently, Ngn3_{cm} levels switch from low to high (OFF-to-ON), and Pdx1_{g} levels toggle from high to low (ON-to-OFF). Additionally, Ngn3_{cm} triggers the transcription of Ngn3_{g} from its genomic promoter, which initiates a positive feedback loop. At high vanillic acid levels (i.e. > 2 µM), VanA₁ is inactivated, and both Ngn3_{cm} and miR30pdx1_{g-shRNA} are shut down. At the same time, the MOR9-1-driven signaling cascade induces the modified high-tightness and lower-sensitivity P_{CREm} promoter that drives the co-cistronic expression of the codon-modified variants of Pdx1 (Pdx1_{cm}) and V-maf musculoaponeurotic fibrosarcoma oncogene homologue A (MafA_{cm}) on pSP17. Consequently, Pdx1_{cm} and MafA_{cm} become fully induced. As Pdx1_{cm} expression ramps up, it initiates a positive feedback loop by inducing the genomic counterparts Pdx1_{g} and MafA_{g}. Importantly, Pdx1_{cm} levels are not affected by miR30Pdx1_{g_shRNA} because the latter is specific for genomic Pdx1_{g} transcripts and because the positive feedback loop-mediated amplification of Pdx1_{g} expression becomes active only after the shutdown of miR30Pdx1_{g-shRNA}. Overall, the differentiation control network provides vanillic acid-programmable, transient, mutually exclusive expression switches for Ngn3 (OFF-ON-OFF) and Pdxl (ON-OFF-ON) as well as the concomitant induction of MafA (OFF-ON) expression, which can be followed in real time.

The expression system of the invention may be used in controlling differentiation, e.g. for differentiating progenitors to pancreatic beta-like cells as it allows for recreating the physiological developmental dynamics in vitro. This may be useful in therapy and/or offers an opportunity to investigate the dosage and timing of transcription factors.

Further provided is a transgenic differentiation-control network comprising the expression system disclosed herein.

The expression system may be encoded by a single isolated nucleic acid or by a plurality of nucleic acids which in their entirety encode the expression system. In a preferred embodiment, such nucleic acid(s) comprise one or more of the group consisting of
(i) an expression cassette for constitutive expression of the MOR9-1 receptor;
(ii) a cAMP-responsive expression cassette for VanA1;
(iii) a vanillic acid responsive expression cassette for Ngn3 and for a regulatory element repressing genomic Pdx 1 expression; and
(iv) a cAMP-responsive expression cassette for Pdxl and for MafA.

Optionally, the Ngn3, Pdxl and/or MafA are codon modified when compared to their respective genomic counterparts.

Also provided is a kit comprising said nucleic acid(s) described above, a mammalian cell line and further instructions for use.

Further provided is a host cell comprising the expression system described above. For example, such host cell may comprise
(i) an expression cassette for constitutive expression of the MOR9-1 receptor;
(ii) a cAMP-responsive expression cassette for VanA1;
(iii) a vanillic acid responsive expression cassette for Ngn3 and for a regulatory element repressing genomic Pdx1 expression; and
(iv) a cAMP-responsive expression cassette for Pdx1 and for MafA.

In a preferred embodiment, said Ngn3, Pdx1 and/or MafA are codon-modified.

The host cell is typically a mammalian cell, preferably a human cell. Non limiting examples of host cells include stem cells, induced pluripotent stem cells, somatic cells (such as liver cells or gallbladder cells), endoderm cells, pancreatic progenitor cells, endocrine progenitor cells or pancreatic beta-like cells. Preferably the host cell is an induced pluripotent stem cell, an endoderm cell or a pancreatic progenitor cell, more preferably an induced pluripotent stem cell.

In one aspect, a method of differentiating a cell is provided, comprising the steps of
1. Introducing the expression system of the present invention into said cell;
2. Adding varying doses of one or more ligands to said cell, thereby triggering the sequential expression of two or more genes of interest and altering the phenotype of said cell.

Typically, the cell is a mammalian cell, preferably a human cell. Non limiting examples of suitable cells include stem cells, induced pluripotent stem cells, somatic cells (such as a liver cell or a gallbladder cell), endodermal cells, pancreatic progenitor cells, an/or endocrine progenitor cells. Preferably the cell is an induced pluripotent stem cell, an endoderm cell or a pancreatic progenitor cell, more preferably an induced pluripotent stem cell. The expression system is introduced using conventional techniques in the art, such as transfection or transformation.

The genes of interest may be selected from the regulatory elements described above, i.e. the group consisting of transcription factors (such as Pdx 1, Ngn3 or MafA, or respective functional fragments thereof), hairpin RNA, micro RNAs, siRNA, aptamers, ribozymes, riboswitches, guide RNAs (CRISPR/Cas9) and antisense RNA.

In a preferred embodiment, the cell is differentiated into a pancreatic beta-like cell.

Typically, the cell comprises the nucleic acid(s) described above. In a preferred embodiment, the cell comprises
(i) an expression cassette for constitutive expression of the MOR9-1 receptor;
(ii) a cAMP-responsive expression cassette for VanA1;
(iii) a vanillic acid responsive expression cassette for codon-modified Ngn3 and for a hairpin RNA targeting genomic Pdxl transcripts; and
(iv) a cAMP-responsive expression cassette for codon-modified Pdxl and codon-modified MafA.

As outlined above, a ligand is typically an exogenous molecule. For example, a ligand may be selected from the group consisting of vanillic acid, tetracycline and erythromycin.

In another aspect, a method of differentiating a mammalian cell into a pancreatic beta-like cell is provided, comprising the steps of
a) Introducing a set of genetic elements into said cell, the set of genetic elements forming a differentiation-control network which allows for an inducible expression pattern of the transcription factors Pdxl, Ngn3 and MafA or functional fragments thereof;
b) Exposing the mammalian cell to conditions which allow for the differentially timed expression of said transcription factors, thereby differentiating the mammalian cell into a pancreatic beta-like cell.

The transcription factors are in some embodiments exogenous transcription factors, i.e. they are provided through transcription of their respective coding sequences in one or more expression cassettes. Thus, a cell may comprise an endogenous (i.e. chromosomal) copy of the coding sequence of a given transcription factor as well as an exogenous copy, provided by the genetic elements. In one embodiment, the endogenous and exogenous coding sequence differ in codon usage.

Said genetic elements forming a differentiation-control network are preferably provided by the nucleic acid described above which encodes the expression system provided herein or by a plurality of nucleic acids which in their entirety encode the expression system as described above. Such nucleic acid(s) may be introduced into the cells via conventional techniques such as transduction or transformation.

The three transcription factors Pdxl, Ngn3, or MafA are known to be major drivers for differentiation into a beta-cell. US 20110280842 discloses a method for reprogramming a cell of endoderm origin wherein the protein expression of at least two transcription factors selected from Pdxl, Ngn3, and MafA is increased to convert a cell of endodermal origin into a pancreatic beta-cell. However, in contrast to the methods disclosed herein, said at least two transcription factors are expressed simultaneously. This has the disadvantage that the differentiation is not precisely regulated. Sharma et al., 2009, Dev Biol. 333:108 report that premature expression of transcription factor like MafA inhibits differentiation. The methods disclosed herein provide for a sequential expression pattern of the three transcription factors which has the advantage of precisely controlling the differentiation to pancreatic beta-like cells. The differential timing of expression of said transcription factors is preferably occurs in three sequential phases, being (i)
A first phase in which expression of Pdx1 is induced, Ngn3 expression is repressed and MafA expression is repressed;
(ii) A second phase in which Pdx1 expression is repressed, Ngn3 expression is induced and MafA expression is repressed;
(iii)A third phase in which Ngn3 expression is repressed and expression MafA and Pdxl is induced.

Differently put, the expression profile for the three transcription factors in the differentiation-control network is OFF-ON-OFF for Ngn3, ON-OFF-ON for Pdxl with the concomitant induction of MafA (OFF-OFF-ON).

In some embodiments, simultaneous programming of endogenous transcription factors is coordinated with the exogenous transcription factors encoded by the one or more expression cassettes as described above.

The cell may be selected from any species, but is typically mammalian and preferably of human origin. Examples of mammalian cell include, without being limited to, a stem cells, induced pluripotent stem cells (IPSCs), somatic cells (such as a liver cell or a gallbladder cell), endoderm cells, pancreatic progenitor cells or endocrine progenitor cells. Preferably the cell is an induced pluripotent stem cell, an endoderm cell or a pancreatic progenitor cell, more preferably an induced pluripotent stem cell. Suitable mammalian cells may be obtained from a healthy subject, from a subject having an increased risk of developing diabetes or from a subject having diabetes.

The method described herein can be practiced in vivo or in vitro. In vitro, cells are cultivated under suitable conditions well known to the skilled person. In some embodiments, said first phase (i) in which Pdx1 is induced, Ngn3 expression is repressed and MafA expression is repressed lasts about 4-6 days, preferably about 5 days. In some embodiments, said second phase (ii) in which Ngn3 is expressed and Pdx1 simultaneous downregulated lasts about 3-5 days, preferably about 4 days. In some embodiments, said third phase (iii) in which Ngn3 is repressed with simultaneous Pdx1 and MafA overexpression lasts about 6-8 days, preferably 7 days. If the expression system of the present invention is used, the expression pattern above is controlled by adding of one or more ligands, such as vanillic acid, to the cells.

Expression levels of Pdxl, Ngn3 and/or MafA may be used to decide when to switch from one phase to the other. In some embodiments, in said first phase (i) the expression levels of Pdxl are such that Nkx6.1 gene expression is activated. In some embodiments, in said second phase (ii) the expression levels of Ngn3 are such, that Pax4, NeuroD and/or Nkx2.2 gene expression is activated and/or Pdxl gene expression is downregulated by at least 2 fold. In some embodiments, in said third phase (iii) the expression levels of Pdx1 and MafA are such that Glut2, Gck, G6PC2, PCSK1 and/or insulin gene expression is activated; and Ngn3 levels are not detectable.

The resulting cell mixture containing pancreatic beta-like cells along with non-differentiated precursor cells may also further be used. However, the method may further comprise the step of isolating pancreatic beta-like cells.

A pancreatic beta-like cell produced by the method described herein expresses and preferably secretes insulin in response to glucose. In one embodiment, the pancreatic beta-like cell has an increased expression of a beta cell marker such as, without being limited to, Nkx6.1, MafA, Ucn3, G6pc2, c-peptide, glucose transporter 2 (Glut2), glucokinase (GCK), prohormone convertase 1/3 (PC1/3), β-cell transcription factors NeuroD and Nkx2.2, by a statistically significant amount relative to the progenitor cell from which the pancreatic beta-like cell was derived. In one embodiment, the pancreatic beta-like cell has a decreased expression of a marker selected from the group consisting of: Amylase (Amy), glucagon, somatostatin/pancreatic polypeptide (SomPP), Ck19, Nestin, Vimentin and Tuji by a statistically significant amount relative to the progenitor cell from which the pancreatic beta-like cell was derived.

In a preferred embodiment, the mammalian cell, the pancreatic beta-like cell or any intermediate thereof comprises the expression system of the present invention. Hence, the mammalian cell, the pancreatic beta-like cell or any intermediate thereof comprises the nucleic acids described above and are consequently the host cells described above. Thus, in some embodiments, the mammalian cell, the pancreatic beta- like cell or any intermediate thereof comprises
(i) an expression cassette for constitutive expression of the MOR9-1 receptor;
(ii) a cAMP-responsive expression cassette for VanA1;
(iii) a vanillic acid responsive expression cassette for Ngn3, optionally being codon-modified, and for a hairpin RNA targeting genomic Pdxl transcripts;
(iv) a cAMP-responsive expression cassette for Pdx1 and MafA, either one or both being optionally codon-modified.

In another aspect, a cell or cell mixture produced by the methods herein are provided.

The cells described herein, such as the host cells described above and/or the cells generated by the methods described herein, in particular fully differentiated cells, may be further processed to produce an implant, an organoid (i.e. a three-dimensional organ-bud grown in vitro) and/or an artificial organ.

Also provided is a microcontainer comprising the cells disclosed herein. Such microcontainer may have any size in a micro or macro range. Typically, such microcontainers are polymer microcapsules or microelectromechanical system-based biocapsules.

The cells of the instant invention, the implant, the organoid, the artificial organ and/or the microcontainer may be transferred into a subject, such as a human being. Said subject may be in need of treatment or a healthy subject, e.g. to study pharmacodynamics and/or pharmacokinetics. Provision of the implant, organoid, artificial organ and/or microcontainer to a subject may further require modulating the subject's immune system. This may e.g. be achieved using drugs or via engineering.

Further provided is a method of treating diabetes, comprising the step of transferring the cells, the implant, the organoid, the artificial organ and/or the microcontainer to a subject in need thereof. Such method may require a surgical step, in particular for transferring the surgical organ.

Further provided is the expression system described herein or the nucleic acid(s) described herein for use in the treatment, such as the treatment of diabetes. Also provided is a method of treating diabetes, comprising the step of introducing the expression system described herein to a subject in need thereof and further administering the one or more ligands in an appropriate dosage regimen to allow for the sequential expression of the encoded proteins. Typically, the nucleic acids described herein are used in such method. Also provided is the use of the expression system of the invention or the nucleic acid(s) described herein for the preparation of a medicament, such as a medicament useful for treating diabetes.

The following are examples of the methods and compositions of the invention. It is understood that various other embodiments may be practiced, given the general description provided above.

### EXAMPLES

### General experimental procedures

Network components. Comprehensive design and construction details for all expression vectors are provided in Table 1. Key plasmids are as follows:
pCI-MOR9-1 encodes constitutively expressed vanillic acid receptor (MOR9-1; P_{hCMV}MOR9-1-pA_{SV40}; Saito et al., 2009);
pSP1 encodes cAMP-responsive expression cassette for the vanillic acid dependent transactivator (VanA₁; P_{CRE}-VanA₁-pA_{SV40}). VanA₁ was PCR-amplified from a cloning vector using OSP1 (5'acgctcgcgatccaccATGGACATGCCGCGCATAAAGCCGG-3') and OSP2 (5'-gctgggccggccCTACCCACCGTACTCGTCAATTCC-3'), restricted with *Nru*I/*Fse*I and cloned into the corresponding sites (*Nru*I/_{Fse}I) of pCK53 (P_{CRE}-VanA₁-pA_{SV40}) (Kemmer et al., J. Control. Release 150, 23-29 (2011)).
pSP12 contains a vanillic acid responsive Ngn3_{cm}, mFT and miR30Pdx_{1g-shRNA} expression unit (pA_{SV40}-Ngn3_{cm}←P_{3VanO2}→mFT-_{miR30Pdx1g-shRNA}-pA_{SV40}).
pSP17 contains a cAMP-responsive dicistronic Pdx1_{cm} and MafA_{cm} expression unit (P_{CREm}-Pdx1_{cm}-2A-MafA_{cm}).

### Table 1: Plasmids and oligonucleotides used and designed herein

Oligonucleotides: restriction endonuclease-specific sites are underlined in oligonucleotide sequences. Annealing base pairs contained in oligonucleotide sequences are shown in capital letters.

Abbreviations: **DsRed-Express,** rapidly maturing variant of the *Discosoma* sp. red fluorescent protein; **EGFP,** enhanced green fluorescent protein; EYFP, enhanced yellow fluorescent protein; **FLuc,** firefly *Photinus pyralis* luciferase; GFP, green fluorescent protein; **GLuc,** *Gaussia princeps* secreted luciferase; **CREₘ,** modified cAMP response element; **MafA_{cm},** codon-modified v-maf musculoaponeurotic fibrosarcoma oncogene homolog A sequence encoding native MafA by a sequence that is distinct from chomosomally encoded MafA; MCS, multiple cloning site; **mFT,** mCherry-derived medium blue-to-red fluorescent timer; **miR30_{Fluc},** microRNA30 containing a firefly luciferase-specific small hairpin RNA; **miR30Pdx1_{g-shRNA},** microRNA30-derived Pdx1_{g}-specific small hairpin RNA; **MOR9-1,** mammalian olfactory receptor 9, a N-terminally rhodopsin-tagged GPCR responsive to vanillic acid; NeuroD, Neurogenic differentiation 1; Ngn3cm, codon-modified neurogenin 3 sequence encoding native Ngn3 by a sequence that is distinct from chromosomally encoded Ngn3; **pA_{bGH},** polyadenylation signal of the bovine growth hormone; **pA_{SV40},** polyadenylation signal of the simian virus 40; **P_{cαA},** mammalian αA-crystallin promoter; **P_{CRE},** synthetic mammalian promoter containing a cAMP-response element (CRE-PhCMVmin); **P_{CREm},** synthetic mammalian promoter containing a modified cAMP-response element (CREₘ-Pₘᵢₙ); **Pdx1_{cm},** codon-modified sequence of the pancreatic and duodenal homeobox 1 encoding native Pdxl protein by a distinct sequence differing from Pdx1_{g}.; **Pdx1_{g},** genomic sequence of the pancreatic and duodenal homeobox 1 encoding native Pdx1 by the wild-type sequence that differs from Pdx1_{cm}; **P_{E1},** mammalian NeuroD E-box promoter; **P_{hCMV},** human cytomegalovirus immediate early promoter; **P_{hCMVmin},** minimal version of human cytomegalovirus immediate early promoter; **P_{hCMV*-1},** tetracycline-responsive promoter (tetO7-P_{hCMVmin}); **P_{hCMVtight},** modified variant of P_{hcMVmin}; **P_{hINS},** human insulin promoter (-881 to +54); **Pₘᵢₙ,** pGL4.23-derived minimal promoter; **P_{SMS900},** mammalian somatostatin promoter; **Pdx1_{UTR},** Pdx1-derived untranslated region; **P_{1VanO2};** vanillic acid-responsive promoter (VanO₂-P_{hCMVmin}); **P_{3VanO2},** vanillic acid responsive promoter (P_{hCMVtigh}-VanO₂-P_{hCMVmin}); **TetR,** Escherichia coli Tn10-derived tetracycline repressor; **TREmod,** Tet response element; **SEAP,** human placental secreted alkaline phosphatase; **tetO₇,** heptameric TetR-specific operator module; **VanA1**; vanillic acid-dependent transactivator (VanR-VP16); **VanOₙ,** synthetic operator module containing n VanR-specific operators; **VanR,** repressor of the *Caulobacter crescentus* VanAB gene cluster; **VP16**, *Herpes simplex* virus protein 16; **2A,** foot and mouth disease virus-derived 2A sequence. **5'/3' LTR**, human immunodeficiency virus long terminal repeat.

| Plasmid | Description and Cloning Strategy | Reference or Source |
|---|---|---|
| pcDNA3.1(+) | Constitutive P_{hCMV}-driven mammalian expression vector (P_{hCMV}-MCS-pA_{bGH}). | Invitrogen |
| pP_{cαA}-FLuc | Mammalian crystallin promoter reporter (P_{cαA}-FLuc-pA_{SV40}). | Yoshida et al., Genes Cells 7, 693-706 (2002) |
| pCMV-GLuc2 | Constitutive GLuc expression vector (P_{hCMV}-GLuc-pA_{SV40}). | NEB |
| pCMV-NeuroD | Constitutive NeuroD expression vector. (P_{hCMV}-NeuroD-pA_{SV40}). | GenBank: BC009046; Image ID: 3873419 |
| pd2EYFP-N1 | Constitutive D2EYFP expression vector (P_{hCMV}-d2EYFP-pA_{SV40}). | Clontech |
| pP_{E1}-FLuc | Mammalian NeuroD promoter reporter (P_{E1}-FLuc pA_{SV40}) | Huang et al., Mol. Cell Biol 20, 3292-3307 (2000) |
| pEYFP-C1 | Constitutive EYFP expression vector (P_{hCMV}-EYFP-pA_{SV40}). | Clontech |
| pEGFP-N1 | Constitutive EGFP expression vector (P_{hCMV}-EGFP-pA_{SV40}). | Clontech |
| pGL4.23 | Expression vector encoding a minimal promoter and FLuc (Pₘᵢₙ-FLuc-pA_{SV40}). | Promega |
| pSEAP2-basic | Mammalian SEAP expression vector lacking promoter and enhancer sequences (MCS-SEAP-pA_{SV40}). | Clontech |
| pSEAP2-control | Constitutive mammalian SEAP expression vector (P_{SV40}-SEAP-pA_{SV40}). | Clontech |
| pP_{SMS900}-FLuc | Mammalian somatostatin promoter reporter (P_{SMS900}-FLuc-pA_{SV40}). | Schwartz et al., Mol Endocrinol 12, 1280-1293 (1998) |
| pTRE-Tight-BI-DsRed-Express | Mammalian expression vector for bidirectional tetracycline-responsive expression of DsRed along with a gene of interest (pA_{SV40}-DsRed-Express ← P_{hCMVmin}-TRE_{mod}-P_{hCMVmin} → MC SpA_{SV40}). | Clontech |
| pUC57 | pUC19-derived prokaryotic expression vector. | Genescript |
| pCI-MOR9-1 | Constitutive expression vector encoding the mammalian vanillic acid receptor MOR9-1 (P_{hCMV}-MOR9-1-pA_{SV40}). | Saito et al., Sci Signal 2, ra9 (2009) |
| pPRIME-TET-GFP-FF3 | Lentiviral expression vector encoding tetracycline-responsive expression of EGFP and the miR30-based shRNA targeting FLuc. (5'LTR-P_{hCMV*-1}-GFP-miR30_{FLuc}-3'LTR). | Stegmeier et al., PNAS USA 102, 13212-13217 (2005) |
| pTRE-Medium-FT | pTRE-derived mammalian expression vector for tetracycline-responsive expression of the medium blue-to-red fluorescent timer (P_{hCMV*-1}-mFT-pA_{SV40}). | Subach et al., Nat Chem Biol 5, 118-126 (2009) |
| pMC1 | Custom-designed pUC57-derived vector containing codon-modified human Ngn3_{cm}. | This work |
| pMC2 | Custom-designed pUC57-derived vector containing codon-modified human Pdx1_{cm}. | This work |
| pMC3 | Custom-designed pUC57-derived vector containing codon-modified human MafA_{cm}. | This work |
| pCK53 | Vector encoding a P_{CRE}-driven SEAP expression unit (P_{CRE}-SEAP-pA_{SV40}). | Kemmer et al., J Control Release 150, 23-29 (2011) |
| pMF111 | Tetracycline-responsive SEAP expression vector (P_{hCMV*-1}-SEAP-pA_{SV40}). | Fussenegger et al., Biotechnol bioeng 55, 927-939 (1997) |
| pMG250 | Constitutive VanA₁ expression vector (P_{SV40}-VanA₁-pA_{SV40}). | Gitzinger et al, Nucleic Acid Res 40, e37 (2012) |
| pMG252 | Vector encoding a VanA₁-specific vanillic acid-responsive P_{1VanO2}-driven SEAP expression unit (P_{1VanO2}-SEAP-pA_{SV40}). | Gitzinger et al, Nucleic Acid Res 40, e37 (2012) |
| pMM44 | Constitutive mammalian GLuc expression vector. GLuc was PCR-amplified from pCMV-GLuc2 using OMM71 (5'-cggaattcaccggtATGGGAGTCAAAGTTCTGTT TG-3') and OMM72 (5'-gaagatctggccggcctct agaTTAGTCACCACCGGCCCCCTTG-3'), restricted with *Eco*RI/*Xba*I and cloned into the corresponding sites (*Eco*RI/*Xba*I) of pSEAP2-control. (P_{SV40}-GLuc-pA_{SV40}). | This work |
| pSP1 | P_{CRE}-driven VanA₁ expression vector. VanA₁ was PCR-amplified from pMG250 using OSP1 (5'-acgctcgcgatccaccATGGACATGCCGCGCATAAAGCCGG-3') and OSP2 (5'-gctgggccgsccCTACCCA CCGTACTCGTCAATTCC-3'), restricted with *Nru*I/*Fse*I and cloned into the corresponding sites (*Nru*I/*Fse*I) of pCK53. (P_{CRE}-VanA₁-pA_{SV40}). | This work |
| pSP2 | P_{hCMV}-driven Ngn3_{cm} expression vector. Ngn3_{cm} was PCR-amplified from pMC1 using OSP3 (5'-acgcgaattccaccATGACCCCCCAGCCAAGCGG AG-3') and OSP4 (5'-acgctctagaTTACAGAAAATCGC TAAAAGCCAG-3'), restricted with *Eco*RI/*Xba*I and cloned into the corresponding sites (*Eco*RI/*Xba*I) of pcDNA 3.1(+). (P_{hCMV}-Ngn3_{cm}-pA_{bGH}). | This work |
| pSP3 | P_{hCMV}-driven Pdx1_{cm} expression vector. Pdx1_{cm} was PCR-amplified from pMC2 using OSP5 (5'-acgcgaattccaccATGAACGGGGAGGAACAGTATTATGC-3') and OSP6 (5'-acgctctagaTTAGCGGGG TTCCTGAGGTCTCCTTG 3'), restricted with *Eco*RI/*Xba*I and cloned into the corresponding sites (*Eco*RI/*Xba*I) of pcDNA 3.1(+). (P_{hCMV}-Pdx1_{cm}-pA_{bGH}). | This work |
| pSP4 | P_{hCMV*-1}-driven MafA_{cm} expression vector. MafA_{cm} was PCR-amplified from pMC3 using OSP5 (5'-acgcgaattcccaccATGGCTGCTGAACTGGCTATG-3') and OSP6 (5'-acgcaagcttTTACAGAAAGAAGTCAGCGGT GCC -3'), restricted with *Eco*RI/*Hin*dIII and cloned into the corresponding sites (*Eco*RI/*Hin*dIII) of pMF111. (P_{hCMV*-1}-MafA_{cm}-pA_{SV40}). | This work |
| pSP5 | P_{hCMV}-driven driven MafA_{cm} expression vector. MafA_{cm} was excised from pSP4 using *Eco*RI/*Not*I and cloned into the corresponding sites (*Eco*RI/*Not*I) of pcDNA 3.1(+). (P_{hCMV}-MafA_{cm}-pA_{bGH}). | This work |
| pSP6 | P_{hCMV*-1}-driven EGFP and miR30Pdx1_{g}-_{shRNA} expression vector. The Pdx1_{g}-specific hairpin oligonucleotide (5'-TGCTGTTGACAGTGAGCGCGGAGTTCCTATTCAACAAGTATAGTGAAGCCA CAGATGTATACTTGTTGAATAGGAACTCC TTGCCTACTGCCTCGGA-3') was PCR-amplified using OSP7 (5'-gatggctgctcgagAAGGTATATTGCTGTTGACA GTGAGCG-3') and OSP8 (5'-gtctagaggaattcCGAGGCAGTAGGCA-3'), restricted with *Xho*I/*Eco*RI and cloned into the corresponding sites (*Xho*I/*Eco*RI) of pPRIME-TET-GFP-FF3. (P_{hCMV*-1}-EGFP-miR30Pdx1_{g- shRNA}-pA_{SV40}). | This work |
| pSP7 | P_{hCMV*-1}-driven Ngn3_{cm} expression vector. Ngn3_{cm} was excised from pSP2 using *Eco*RI/*Xba*I and cloned into the corresponding sites (*Eco*RI/*Xba*I) of pTRE-Tight-BI-DsRed-Express. (pA_{SV40}-Ngn3_{cm}←P_{hCMVmin}-TRE_{mod}-P _{hCMVmin}→ MCS-pA_{SV40}). | This work |
| pSP8 | P_{1VanO2}-driven EGFP and miR30Pdx1_{g-shRNA} expression vector. P_{1VanO2} was PCR-amplified from pMG252 using OSP9 (5'-acgctctagaGTCAATTCGCGAATTGGATCCAATAGCG-3') and OSP10 (5'-gctaaccggtC GCGGAGGCTGGATCGG-3'), restricted with *Xba*I/*Age*I and cloned into the corresponding sites (*Xba*I/*Age*I) of pSP6. (P_{1VanO2}-GFP-miR30Pdx1_{g-shRNA}-pA_{SV40}). | This work |
| pSP10 | P_{1VanO2}-driven mFT and miR30Pdx1_{g-shRNA} expression vector. mFT was PCR-amplified from pTRE-Medium-FT using OSP13 (5'-gcatgaattcaccggtcgccacc ATGGTGAGCAAGGGCGAGGAGGATAAC-3') and OSP14 (5'-gcattctaga gcggccgcTTACTTGTACAGCTCGTCCATG-3'), restricted with (*Age*I/*Not*I) and cloned into the corresponding sites (*Age*I/*Not*I) of pSP8. (P _{1VanO2}-mFT-miR3 0Pdx 1 _{g-shRNA}-pA_{SV40}). | This work |
| pSP12 | P_{3VanO2}-driven Ngn3_{cm}, mFT and miR30Pdx1_{g-shRNA} expression vector. P_{1VanO2}-mFT-mir30Pdx1_{g-shRNA} was PCR-amplified from pSP10 using OSP15 (5'-acgcctcgagGTCAATTCGCGAATTGGATCCAATAGCG-3') and OSP16 (5'-acgcaagcttCGCGTCGCCGCGTGTTTAAACGCATTAG-3'), restricted with *Psp*XI/*Hind*III and cloned into the corresponding sites (*Psp*XI/*Hind*III) of pSP7. (pA_{SV40} - Ngn3_{cm}←P_{3VanO2}→mFT-miR30Pdx 1 _{g-shRNA}-pA_{SV40}). | This work |
| pSP14 | SEAP2-control-based expression vector encoding a miR30Pdx1_{g-shRNA}-sensitive SEAP transcription unit linked to Pdx1_{UTR}. SEAP was PCR-amplified from pSEAP2-control using OSP17 (5'- acgcgaattcGCCCACCATGCTGC-3') and OSP18 (5'- acgctctaga tacttgttgaataggaactccttTCATGTCTGCTCGAAGCGGCCGGCCGCCCCGACTCTTG-3'), restricted with *Eco*RI/*Xba*I and cloned into the corresponding sites (*Eco*RI/*Xba*I) of pSEAP2-control. (P_{SV40} -SEAP-Pdx1_{UTR}-pA_{SV40}). | This work |
| pSP15 | pSEAP2-basic containing CREm. (CREm-SEAP-pA_{SV40}). | |
| pSP16 | pSEAP2-basic containing a P_{CREm}-driven SEAP expression unit. (P_{CREm}-SEAP-pA_{SV40}). | |
| pSP17 | Dicistronic P_{CREm}-driven Pdx1_{cm} and MafA_{cm} expression vector. Pdx1_{cm}-2A was PCR-amplified from pSP3 using OSP5 (5'-acgcgaattccaccATGAACGGGGAGGAACAGTATTATGC-3') and OSP22 (5'-aggtccagggttggactccacgtctcccgccaacttgagaaggtca aaattcaacaaGCGGGGTTCCTGAGGTCTCCTTG-3'). 2A-MafA_{cm} was PCR-amplified from pSP5 using OSP23 (5'-ttgttgaattttgaccttctcaagttggcgggagacgtggagtc caaccctggacctATGGCTGCTGAACTGGCTATG -3') and OSP24 (5'-gcatgcgcgctctagattaCAGAAAGAAGTCAGCGGTGCC-3'). Both PCR fragments were annealed and Pdx1_{cm}-2A-MafA_{cm} was PCR-amplified using OSP5 and OSP24, restricted with *Eco*RI/*Bss*HII and cloned into the corresponding sites (*Eco*RI/*Bss*HII) pSP16. (P_{CREm}-Pdx1_{cm}-2A-MafA_{cm}-pA_{SV}40). | This work |
| pSP19 | pUC57 containing the human insulin promoter (P_{hINS}; -881 to +54). | This work |
| pSP21 | P_{hINS}-driven GLuc expression vector. P_{hINS} was excised from pSP19 with *Xho*I/*Eco*RI and cloned into the corresponding sites (*Xho*I/*EcoR*I) of pMM44. (P_{hINS}-GLuc-pA_{SV40}), | This work |
| pSP24 | P_{CREm}-driven EYFP expression vector. P_{CREm} was excised from pSP15 with *Mlu*I/*Eco*RI and EYFP was PCR-amplified from pEYFP-Clusing OMM48 (5'-ggaattcactagtgcccggggaaccggtATGGTGAGCAAGGGCGAG-3') and OMM54 (5'-gctctagatctggccggccctaTTACTTGTACAGCTCGTCCATG-3'), restricted with *Eco*RI/*Xba*I and both fragments were cloned into the compatible sites (*MluI*/*Xba*I) of pSEAP2-basic. (P_{CREm}-EYFP-pA_{SV40}). | This work |
| pSP25 pSP26 | Dicistronic P_{hCMV}-driven Pdx1_{cm}-2A-MafA_{cm} expression vector. Pdx1_{cm}-2A-MafA_{cm} was excised from pSP17 with *Eco*RI/*Xba*I and cloned into the corresponding sites (*Eco*RI/*Xba*I) of pcDNA3.1(+). (P_{hCMV}-Pdx1_{cm}-2A-MafA_{cm}-pA_{SV40}). P_{hINS}-driven DsRed-Express expression vector. DsRed-Express was PCR-amplified from pTRE-Tight-BI-DsRed-Express using OSP25 (5'-acgcgaattcgccaccATGGCCTCCTCCGAGGACGTC-3') and OSP26 (5'-acgctctagaCTACAGGAACAGGTGGTGGCG-3'), restricted with (*EcoRI*/*XbaI*) and cloned into the corresponding sites (*EcoR*I/*XbaI*) of pSP21. (P_{hINS}-DsRed- Express-pA_{SV40}). | This work This work |

Cell culture and transfection: Culturing methods are described in Pagliuca FW, et al., Cell 159, 428-439 (2014), and Rezania A, et al., Nat Biotechnol 32, 1121-1133 (2014). Human mesenchymal stem cells transgenic for the catalytic subunit of human telomerase (hMSC-TERT) were cultivated in Dulbecco's modified Eagle's medium (DMEM; Invitrogen, Lucerne, Switzerland) supplemented with 10% (v/v) foetal calf serum (FCS; lot no. PE01026P, Bioconcept, Allschwil, Switzerland) and 1% (v/v) penicillin/streptomycin solution (Sigma-Aldrich, Buchs, Switzerland) at 37°C in a humidified atmosphere containing 5% CO₂.

The human induced pluripotent stem cells (hIPSCs) were derived from the adipose tissue of a 50-year-old donor, as described in Heng BC, et al. Metab Eng 18, 9-24 (2013). The hIPSCs were cultivated in Geltrex™-coated 12-well culture plates (Invitrogen) containing 1 mL mTeSR1™ medium (STEMCELL Technologies, Grenoble, France). For serial passage, the colonies were enzymatically dissociated into cellular clumps (200 to 400 cells per clump) using 1 U/mL dispase (STEMCELL Technologies).

For the transfection of hMSC-TERT, 2x10⁵ cells were seeded per well on a 6-well plate 12 h prior to transfection and incubated for 24 h with 400 µL of a DNA-PEI mixture that was produced by incubating 6 µL PEI (PEI, <20000 MW, Polysciences, Eppelheim, Germany; stock solution: 1 mg/mL ddH₂O, pH 7.2) with 2 µg of total DNA, vortexing for 5 s and incubating for 15 min at 22°C. Prior to transfection, the hIPSCs-derived pancreatic progenitor cells were dissociated using 0.5 mL of StemPro® Accutase® Cell Dissociation Reagent (Invitrogen) and reseeded into 12-well plates. For the transfection, 3 µg of total DNA was transfected into cells cultivated in each well of a 12-well plate using Lipofectamine® LTX Reagent with PLUS™ Reagent (Invitrogen). The plasmids (pCI-MOR9-1; pSP1; pSP12; pSP17) were cotransfected at a ratio of 1:1:1:1 for hIPSCs (1:0.01:1:1 for hMSC-TERT). The transfection efficiency was determined via FACS analysis using pEGFP-N1-transfected, randomly differentiating cells as a control (Fig. 19).

For sorting the insulin positive cells, differentiated cells (day 11) were prepared for transfection by dissociating the cells with 0.5 mL of StemPro® Accutase® Cell Dissociation Reagent (Invitrogen), seeded into the wells of a 12-well plate and cultivated for 4 h in 1 mL RPMI (Invitrogen) containing 10% FCS and supplemented with 10 µM Y-27632, 11 mM glucose, 400 µM vanillic acid (Sigma-Aldrich), 50 ng/mL exendin-4 (Tocris Bioscience), 10 mM nicotinamide (Sigma-Aldrich) and 40 µM beta-mercaptoethanol. Subsequently, the culture medium was replaced by the same supplemented RPMI devoid of Y-27632 and the cells were transfected with 3 µg pSP26 (P_{hINS}-DsRed-Express-pA_{SV40}) using Lipofectamine® LTX with Plus™ Reagent (Invitrogen).

For sorting the transfected insulin positive cells using the growth factor/chemical-based method on day 11, hIPSCs-derived pancreatic progenitor cells (day 0) were transfected with (pCI-MOR9-1; pSP1; pSP12; pSP17; pEGFP-N1; pSP26) for differentiation-control network and (pEGFP-N1; pSP26) for control. The parental vector pcDNA3.1(+) (Invitrogen) was used as a filler plasmid when replacing functional components for control purposes.

For the dose response experiments, the transfected cells were trypsinised (200 µL trypsin, 5 min, 37°C; PAN Biotech, Aidenbach, Germany) and transferred into the wells of a 96-well plate (104 cells/well) containing 100 µL DMEM supplemented with different concentrations of vanillic acid. SEAP expression was profiled after 48 h.

Differentiation of hIPSCs. The hIPSCs were differentiated to human pancreatic progenitor cells and pancreatic beta-like cells as described previously (see Pagliuca FW, et al., Cell 159, 428-439 (2014), Rezania A, et al., Nat Biotechnol 32, 1121-1133 (2014), Russ HA, et al. EMBO J 34, 1759-1772 (2015), Nostro MC, et al., Development 138, 861-871, (2011), Cheng X, et al. Cell Stem Cell 10, 371-384 (2012)). For the growth factor/chemical-based method, suspension 12-well plates were used from Day 0 onwards; Noggin and GSI (Gamma secretase inhibitor) were removed to prevent precocious endocrine differentiation prior to the induction of human pancreatic progenitors expressing the transcription factors Pdxl and Nkx6.135. Subsequently EGF (Epidermal growth factor) was added to trigger the expression of Nkx6.1 in pancreatic progenitor cells35. The transfected cells were allowed to form aggregates in presence of thyroid hormone T3 (Triiodothyronine) and retinoic acid to induce endocrine differentiation and then subsequent maturation was triggered using thyroid hormone T3 (Triiodothyronine) and Alk5 inhibitor (Alk5i). Roswell Park Memorial Institute (RPMI) 1640 culture medium, Iscove's modified Dulbecco's medium (IMDM), Ham's F12 nutrient mixture, B-27® serum-free supplement, N-2 supplement, human basic fibroblast growth factor (bFGF) and KnockOutTM Serum Replacement (KOSR) were purchased from Invitrogen. Human bone morphogenetic protein 4 (BMP4), the sonic hedgehog antagonist KAAD-Cyclopamine, human Noggin, human Epidermal growth factor (EGF), human fibroblast growth factor 7/10 (FGF-7/FGF-10) and human vascular endothelial growth factor (VEGF) were obtained from Miltenyi Biotec (Bergisch Gladbach, Germany). Human Activin A, wingless-type MMTV integration site family member 3A (Wnt3A), vanillic acid, ascorbic acid, bovine serum albumin (BSA), 1-thioglycerol, thyroid hormone T3 and retinoic acid were purchased from Sigma-Aldrich. The gamma secretase inhibitor L685,458 was obtained from Tocris Bioscience (Bristol, UK), Rock inhibitor Y-27632 was obtained from STEMCELL Technologies (Grenoble, France) and Alk5 inhibitor was obtained from Enzo lifesciences (Lausen, Switzerland).

Immunocytochemistry. At different time points of the differentiation (endoderm; pancreatic progenitor, endocrine progenitor cells; pancreatic beta-like cells), differentiation-control network-transgenic hIPSCs cultivated in 12-well plates were fixed with 4% (w/v) paraformaldehyde solution overnight at 4°C. The fixed cells were permeabilised with 0.1% (v/v) Triton X-100 solution and then incubated with blocking solution (10% (v/v) goat serum in PBS) for 1 h at 37°C followed by DAPI-mediated staining of the cell nuclei (Vectashield®; Vector Labs, Burlingame, CA, USA). The samples were immunostained for 1 h at 37°C using primary antibodies specific for Sox17 and Fox A2 (endoderm), Pdx1 (pancreatic progenitor), Ngn3, Pdxl and VP16 (endocrine progenitor cells) and C-peptide, VP16, Pdxl and MafA (pancreatic beta-like cells), washed three times in 0.1% Tween-20 and incubated for 1 h with appropriate fluorophore-conjugated secondary antibodies before immunofluorescence was visualised with a Nikon Eclipse Ti microscope (Nikon AG, Zurich, Switzerland) using the appropriate excitation/emission wavelengths. The primary antibodies utilised in this study included mouse anti-Sox17 IgG (MAB19241, lot no. C614013; 10 µg/mL; R&D Systems Europe, Abingdon, UK), rabbit anti-FoxA2 IgG (AB4125, lot no. NMM1757191; dilution 1:1000; Millipore, Billerica, MA, USA), rabbit anti-VP16 IgG (ab4808, lot no. 830675; dilution 1:200; Abcam, Cambridge, UK), mouse anti-Ngn3 IgG (F25A1B3, lot no. GR95038; dilution 1:100; Developmental Studies Hybridoma Bank, Iowa City, IA, USA), mouse anti-Pdxl IgG (MAB2419, lot no. C267712; dilution 1:2000; R&D Systems, Europe), rabbit anti-MafA IgG (sc-66958, lot no. C1609; dilution 1:100; Santa Cruz Biotechnology) and rat anti-C-peptide IgG (GN-ID4, lot no. 10510; dilution 1:50; Developmental Studies Hybridoma Bank). The secondary antibodies utilised in this study included fluorescein (FITC)-conjugated goat anti-mouse IgG (AP124F, lot no. LV1688510; dilution 1:1000) and rhodamine conjugated goat anti-rabbit IgG (AP307R, lot no. LV1441875; dilution 1:1000) from Millipore Inc. and fluorescein (FITC)-conjugated chicken anti-rat IgG (sc-2991, lot no. K2912; dilution 1:400) from Santa Cruz Biotechnology.

Analytical assays. SEAP: The production of human placental secreted alkaline phosphatase (SEAP) was quantified in cell culture supernatants using a p-nitrophenylphosphate-based light absorbance time course. GLuc: Activity of Gaussia princeps Luciferase (GLuc) was quantified using a BioLux® Gaussia Luciferase Assay Kit, (New England Biolabs Inc., USA). FLuc: The activity of Photinus pyralis firefly luciferase (FLuc) was quantified using a Luciferase Assay System (Promega, Dübendorf, Switzerland).

Quantitative RT-PCR: Total RNA of differentiation-controlled hIPSC population and FACS-sorted hIPSC-derived pancreatic beta-like cells was isolated using the ZR RNA MiniPrepTM kit (Zymo Research, Irvine, CA, USA) and TURBOTM DNase (Invitrogen). qRT-PCR with total RNA of differentiation -controlled hIPSC population was performed using SuperScript® II Reverse Transcriptase (Invitrogen) and TaqMan® Fast Advanced Mastermix (Invitrogen) with the corresponding TaqMan® Gene Expression Assays (see Table 2) or SYBR® Green PCR Mastermix with custom-designed primers (SEQ ID NOs. 1 - 26). qRT-PCR with total RNA of FACS sorted hIPSC-derived pancreatic beta-like cells was performed using High-Capacity cDNA Reverse Transcription Kit (Invitrogen) and Taqman® PreAmp Mastermix Kit (Invitrogen) with the corresponding TaqMan® Gene Expression Assays (Table 2). The Eppendorf Realplex Mastercycler (Eppendorf GmbH, Hamburg, Germany) was set to the following amplification parameters: 2 min at 50°C, 20 s at 95°C and 40 cycles of 1 s at 95°C followed by 1 min at 60°C. The relative threshold cycle (Ct) was determined and normalised to the endogenous GAPDH transcript. The fold change for each transcript relative to the control was calculated using the comparative Ct method (see Schmittgen TD, Livak KJ. Nat Protoc 3, 1101-1108 (2008)).

**Table 2: TaqMan® Gene Expression Assays**

| Gene | Gene ID | Assay ID | Dye |
|---|---|---|---|
| Abcc8 | 6833 | Hs01093761_m1 | FAM |
| Acox2 | 8309 | Hs00185873_m1 | FAM |
| Cacna1D | 776 | Hs01073321_m1 | FAM |
| Chgb | 1114 | Hs01084631_m1 | FAM |
| Ck19 | 3880 | Hs00761767_s1 | FAM |
| Dl11 | 28514 | Hs00194509_m1 | FAM |
| Dpp4 | 1803 | Hs00897391_m1 | FAM |
| FoxA1 | 3169 | Hs04187555_m1 | FAM |
| FoxA2 | 3170 | Hs00232764_m1 | FAM |
| Fzd2 | 2535 | Hs00361432_s1 | FAM |
| Gapdh | 2597 | Hs02758991_g1 | FAM |
| Gcgr | 2642 | Hs01026189_g1 | FAM |
| Gck | 2645 | Hs01564555_m1 | FAM |
| Ghrelin | 51738 | Hs01074053_m1 | FAM |
| Glis3 | 169792 | Hs00541450_m1 | FAM |
| Glucagon | 2641 | Hs01031536_m1 | FAM |
| Glut2 | 6514 | Hs01096904_m1 | FAM |
| G6PC2 | 57818 | Hs01549773_m1 | FAM |
| Hes1 | 3280 | Hs00172878_m1 | FAM |
| lapp | 3375 | Hs00169095_m1 | FAM |
| Insulin | 3630 | Hs02741908_m1 | FAM |
| Irx2 | 153572 | Hs01383002_m1 | FAM |
| Kcnj 11 | 3767 | Hs00265026_s1 | FAM |
| Kcnk1 | 3775 | Hs00158428_m1 | FAM |
| Kcnk3 | 3777 | Hs00605529 _ml | FAM |
| MafA | 389692 | Hs01651425_s1 | FAM |
| MafB | 9935 | Hs00534343_s1 | FAM |
| Mmp2 | 4313 | Hs01548727_m1 | FAM |
| Mnx1 | 3110 | Hs00907365_m1 | FAM |
| NeuroD1 | 4760 | Hs01922995_s1 | FAM |
| Ngn3 | 50674 | Hs01875204_s1 | FAM |
| Nkx6.1 | 4825 | Hs00232355_m1 | FAM |
| Onecut2 | 9480 | Hs00191477_m1 | FAM |
| Pax4 | 5078 | Hs00173014_m1 | FAM |
| Pcskl | 5122 | HsOI026107_m1 | FAM |
| Pcsk2 | 5126 | Hs01037347_m1 | FAM |
| Sftpd | 6441 | Hs01108490_m1 | FAM |
| Pdx1 | 3651 | Hs00236830_m1 | FAM |
| Slc30a8 | 169026 | Hs00545183_m1 | FAM |
| Snap25 | 6616 | Hs0093962_m1 | FAM |
| Stxbp1 | 6812 | Hs01119036_m1 | FAM |
| Stx1A | 6804 | Hs00270282_m1 | FAM |
| Somatostatin | 6750 | Hs00356144_m1 | FAM |
| Sox17 | 64321 | Hs00751752_s1 | FAM |
| Syt4 | 6860 | Hs01086433_m1 | FAM |
| Ucn3 | 114131 | Hs00846499_s1 | FAM |

Fluorescence-activated cell sorting of differentiated and transfected cells. Pancreatic beta-like cells (day 14) were sorted using a Becton Dickinson LSRII Fortessa flow cytometer (Becton Dickinson, Allschwil, Switzerland) equipped for DsRed (561 nm laser, 505 nm long-pass filter, 586/15 emission filter) detection and pancreatic beta-like cells differentiated using growth factor/chemical-based method (day 11) were sorted using DsRed (561 nm laser, 505 nm long-pass filter, 586/15 emission filter) detection and FITC/EGFP (488 nm laser, 505 nm long-pass filter, 530/30 emission filter) detection and set to exclude dead cells and cell doublets. Total RNA extracted from the sorted pancreatic beta-like cells was used for qRT-PCR. For FACS analysis, we used the same antibodies and dilutions as for immunostaining. The hIPSCs (day - 10) differentiated for 5 (day -6) and 11 (day 0) days and pEGFP-N1-transfected human pancreatic progenitor cells (day 0) were detached (1x10⁶ cells/mL) using 0.5 mL StemPro® Accutase® Cell Dissociation Reagent (Invitrogen). The cells (1x10⁶ cells/mL) were fixed with 4% (w/v) paraformaldehyde in PBS overnight at 4°C, permeabilised for 10 min with 0.1% (v/v) Triton X-100, washed once in 2 mL PBS and sequentially labelled for 1 h at 37°C with specific primary and secondary antibodies, with a PBS washing step in between. The cell populations were analysed using a Becton Dickinson LSRII Fortessa flow cytometer (Becton Dickinson, Allschwil, Switzerland) equipped for FITC/EGFP (488 nm laser, 505 nm long-pass filter, 530/30 emission filter) detection and set to exclude dead cells and cell doublets.

Electron microscopy. Pancreatic beta-like cells differentiated using the differentiation-control network (day 11) were dissociated into single cells with 0.5 mL of StemPro® Accutase® Cell Dissociation Reagent (Invitrogen) and prepared for electron microscopic analysis using the procedure of Russ HA, et al. EMBO J 34, 1759-1772 (2015).

Quantification of glucose responsiveness. After 11 days of differentiation, growth factor/chemical based differentiation technique derived cells and differentiation-control network (pCI-MOR9-1/pSPI/pSP12/pSP17) -derived cells or human islets were washed in 0.25 mL Krebs-Ringer Bicarbonate Buffer (129 mM NaCl, 5 mM NaHCO₃, 4.8 mM KCl, 1.2 mM KH₂PO₄, 1.2 mM MgSO₄, 2.5 mM CaCl₂, 10 mM HEPES, pH 7.4; Sigma-Aldrich) and incubated for 30 min in low-glucose (Sigma Aldrich) (2.8 mM) Krebs-Ringer Bicarbonate Buffer supplemented with 100 µM 3-isobutyl-1-methylxanthine (IBMX; Sigma-Aldrich). The culture was then switched to medium glucose (10mM) for 30 min and then high-glucose (20 mM) Krebs-Ringer Bicarbonate Buffer for another 30 min. Thereafter, the culture was switched to high-glucose (20 mM), KCl (30 mM) supplemented Krebs-Ringer Bicarbonate Buffer for another 30 min. The secreted isoforms of the connecting peptide (C-peptide) produced during proinsulin processing were quantified using the ultrasensitive human C-peptide ELISA (lot no. 21899; Mercodia, Uppsala, Sweden). The values were normalised to the total intracellular protein content and number of insulin positive cells using a Bio-Rad protein assay (Bio-Rad Laboratories, Hercules, CA, USA).

### Example 1 - Design of a vanillic acid-programmable differentiation-control network

Figure 2a schematically shows an exemplary differentiation control network programming differential expression dynamics of pancreatic transcription factors. The constitutively expressed, vanillic acid-sensitive olfactory G protein-coupled receptor MOR9-1 on the expression vector pCI-MOR9-1 senses extracellular vanillic acid levels and triggers a synthetic signalling cascade, inducing PCRE-driven expression of the transcription factor VanA₁, expressed from pSP1. At medium vanillic acid concentrations, VanA₁ binds and activates the bidirectional vanillic acid-responsive promoter P_{3VanO2} on pSP12, which drives the induction of codon modified Neurogenin 3 (Ngn3_{cm}) as well as the coexpression of both the blue-to-red medium fluorescent timer (mFT) for precise visualisation of the unit's expression dynamics and a small hairpin RNA programming the exclusive destruction of genomic pancreatic and duodenal homeobox 1 (Pdx1_{g}) transcripts (miR30pdx1_{g-shRNA}). Consequently, Ngn3_{cm} levels switch from low to high (OFF-to-ON), and Pdx1_{g} levels toggle from high to low (ON-to-OFF). Additionally, Ngn3_{cm} triggers the transcription of Ngn3_{g} from its genomic promoter, which initiates a positive feedback loop. At high vanillic acid levels, VanA₁ is inactivated, and both Ngn3_{cm} and miR30pdx1_{g-shRNA} are shut down. At the same time, the MOR9-1-driven signalling cascade induces the modified high-tightness and lower-sensitivity P_{CREm} promoter that drives the co-cistronic expression of the codon-modified variants of Pdx1 (Pdx1_{cm}) and V-maf musculoaponeurotic fibrosarcoma oncogene homologue A (MafA_{cm}) on pSP17. Consequently, Pdx1_{cm} and MafA_{cm} become fully induced. As Pdx1_{cm} expression ramps up, it initiates a positive feedback loop by inducing the genomic counterparts Pdx1_{g} and MafA_{g}. Importantly, Pdx1_{cm} levels are not affected by miR30Pdx1_{g-shRNA} because the latter is specific for genomic Pdx1_{g} transcripts and because the positive feedback loop-mediated amplification of Pdx1_{g} expression becomes active only after the shutdown of miR30Pdx1_{g-shRNA}. Overall, the differentiation control network provides vanillic acid-programmable, transient, mutually exclusive expression switches for Ngn3 (OFF-ON-OFF) and Pdxl (ON-OFF-ON) as well as the concomitant induction of MafA (OFF-ON) expression, which can be followed in real time.

For the design of a differentiation-control network, constitutive MOR9-1 expression and P_{CRE}-driven VanA₁ expression were combined with pSP12 (pA_{SV40}-Ngn3_{cm}←P_{3VanO2}→mFT-_{miR30Pdx1g}-_{shRNA}-pA_{SV40}) for endocrine specification and pSP17 (P_{CREm}-Pdx1_{cm}-2AMafA_{cm}-pA_{SV40}) for maturation of developing β-cells. The pSP12-encoded expression unit enables the VanA₁-controlled induction of the optimised bidirectional vanillic acid-responsive promoter (P_{3VanO2}) that drives expression of a codon-modified Ngn3_{cm}, the nucleic acid sequence of which is distinct from its genomic counterpart (Ngn3_{g}) to allow for qRT-PCR based discrimination. In the opposite direction, P_{3VanO2} transcribes miR30Pdx1_{g-shRNA}, which exclusively targets genomic Pdxl (Pdx1_{g}) transcripts for RNA interference-based destruction and is linked to the production of a blue-to-red medium fluorescent timer (mFT) for precise visualisation of the unit's expression dynamics in situ. pSP17 contains a dicistronic expression unit in which the modified high-tightness and lower-sensitivity P_{CREm} promoter drives co-cistronic expression of Pdx1_{cm} and MafA_{cm}, which are codon-modified versions producing native transcription factors that specifically differ from their genomic counterparts (Pdx1_{g}, MafA_{g}) in their nucleic acid sequence.

The vanillic acid-controlled expression and functionality of all network components were tested individually. For testing activation of the human NeuroD promoter by Ngn3_{cm} and NeuroD. 2x10⁵ hMSC-TERT were cotransfected with the firefly luciferase (FLuc) reporter construct pP_{E1}-FLuc (P_{E1}-FLuc pA) and either the Ngn3_{cm}-encoding pSP2 (P_{hCMV}-Ngn3_{cm}-pA), the NeuroD containing pCMV-NeuroD (P_{hCMV}-NeuroD-pA) or pcDNA3.1(+) as negative control and grown for 48h before intracellular luciferase was quantified. Mean data ± S.D., n=3, are shown in Figure 5. Activation of the human somatostatin and crystallin promoters by Pdx1_{cm} and MafA_{cm} was tested by cotransfecting 2x10⁵ hMSC-TERT with the constitutive dicistronic Pdx1_{cm} and MafA_{cm} expression vector pSP25 or pcDNA3.1(+) as negative control and either of the somatostatin reporter construct pPSMS900-FLuc (P_{SMS900}-FLuc-pA) or the crystallin reporter vector pPcaA-FLuc (PcaA-FLuc-pA) and grown for 48h before intracellular luciferase was quantified. Figure 6 shows the results; data are mean ± S.D., n=3. Finally, expression levels of Ngn3_{cm} and miR30Pdx1_{g-shRNA} in the presence of medium and high vanillic acid concentrations were quantified. 2x10⁵ hMSC-TERT were cotransfected with differentiation -control vectors pCI-MOR9-1, pSP1 and pSP12) as well as either the NeuroD promoter-driven firefly luciferase (FLuc)-encoding reporter (PE1-FLuc) or the miR30Pdxl_{g-shRA}-sensitive SEAP reporter construct pSP14 (PSV40 -SEAP-Pdx1UTR-pA) and grown for 48h in the presence of medium (2µM) or high (400µM) vanillic acid (VA) concentrations before intracellular luciferase and secreted SEAP was profiled. Results are shown in Figure 7; data are mean ± S.D., n=3.

Subsequently, the differentiation control network was ready to be transfected into hIPSC-derived human pancreatic progenitor cells. These cells are characterized by high expression of Pdx1_{g} and Nkx6.1 levels and the absence of Ngn3_{g} and MafA_{g} production (day 0: Figures 8-13).

Following the transient cotransfection of pCI-MOR9-1 (P_{hCMV}-MOR9-1-pA_{SV40}), pSP1 (P_{CRE}-VanA₁-pA_{SV40}), pSP12 (pA_{SV40}-Ngn3_{cm}←P_{3VanO2}→mFT-miR30Pdx1_{g-shRNA}-pA_{SV40}) and pSP17 (P_{cREm}-Pdx1_{cm}-2A-MafA_{cm}-pA_{SV40}) into hIPSC-derived human pancreatic progenitor cells, the differentiation control network overrides random endogenous differentiation activities and executes the pancreatic beta-cell-specific differentiation program in a vanillic acid remote-controlled manner. To confirm that the differentiation control network operates as programmed, network-transgenic and pEGFP-N1-transfected (negative-control) cells were cultivated for four days at medium (2 µM) and then seven days at high (400 µM) vanillic acid concentrations and profiled the differential expression dynamics of all of the differentiation-control network components and their genomic counterparts as well as the interrelated transcription factors and hormones in both whole populations and individual cells (Fig. 2, 14) at days 0, 4, 7,11 and 14.

### Example 2 - Validation of the differentiation-control dynamics

The aforementioned detailed analysis demonstrated that the following three expression protocols were simultaneously executed:
(i) Positive band-pass Ngn3 expression profile (OFF-ON-OFF): medium vanillic acid concentrations trigger the MOR9-1-based designer cascade and induce P_{CRE}-driven VanA₁ expression. Because VanA₁ remains fully active at medium vanillic acid levels, it transactivates the bidirectional promoter P_{3VanO2} and induces Ngn3_{cm} expression (Figure 2c).
   Ngn3_{cm} triggers the transcription of Ngn3g from its genomic promoter, which initiates a positive feedback loop (Shih et al., Development 139, 2488-2499 (2012)), resulting in high-level expression of Ngn3_{cm/g} (OFF-ON; (Fig. 2c,d, Fig. 14) as well as its target genes Pax4 (paired box gene 4) and the notch signalling components Hes1 (hairy and enhancer of split-1) and Dill (delta-like 1), which manage lateral inhibition (a developmental pathway suppressing similar differentiation of neighbouring cells) (day 4; Fig. 14). NeuroD1 (neurogenic differentiation factor 1) expression levels in differentiation-control network-transfected cells remained identical to randomly differentiating cells (days 4; Fig. 14). After switching the cells to high vanillic acid concentrations, VanA₁ was released from P_{3VanO2} and Ngn3_{cm}, and Ngn3_{g} expression halted, which resulted in an overall decrease of this transcription factor (ON-OFF; day 11; Fig. 2c,d).
(ii) Negative band-pass Pdx1 expression profile (ON-OFF-ON): Following the MOR9-1-mediated activation of P_{CRE}-driven VanA₁ expression at medium vanillic acid concentrations, VanA₁ co-induces the P_{3VanO2}-driven expression of Ngn3_{cm} and miR30Pdx1_{g-shRNA}. As Ngn3_{cm} and miR30Pdx1_{g-shRNA} levels increase, miR30Pdx1_{g-shRNA} programs the exclusive destruction of genomic Pdx1 (Pdx1_{g}) transcripts, which results in a reduction of Pdx1_{g} levels (ON-OFF; Fig. 2d). Because P_{CREm}-driven Pdx1_{cm}-2A-MafA_{cm} transcription is silent at medium vanillic acid concentrations, the overall cellular Pdx1_{cm/g} content remains low (day 4; Fig. 2c,d,e). At high vanillic acid concentrations, where VanA₁ is inactivated and P_{3VanO2}-driven miR30Pdx1_{g-shRNA} expression is shut down, P_{CREm}-mediated Pdx1_{cm} expression ramps up and initiates a positive feedback loop by inducing Pdx1_{g} as well MafA_{g} from their genomic promoters, which results in sustained high-level Pdx1_{g} and MafA_{g} expression (OFF-ON; day 11; Fig. 2d). Importantly, Pdx1_{cm} levels are not affected by miR30Pdx1_{g-shRNA}, as it is specific for genomic Pdx1_{g} transcripts, and the positive feedback loop-mediated amplification of Pdx1_{g} expression becomes active only after the shutdown of miR30Pdx1_{g-shRNA} (Fig. 15). The transition between miR30Pdx1_{g-shRNA}-mediated knock down and P_{CREm}-driven Pdx1_{cm} ramp-up requires a time delay in the production of both components that results from the combination of the higher vanillic acid sensitivity and induction kinetics of P_{CRE} compared with P_{CREm} (Fig. 16) and the fact that miR30Pdx1_{g-shRNA} is driven by a two-level cascade (P_{CRE} inducing VanA₁; VanA₁ activating P_{3VanO2}), which then leads to feed-forward loop-type signal amplification and increased expression kinetics of miR30Pdx1_{g-shRNA} compared with Pdx1_{cm} (Fig. 15). The time delay and correlating expression dynamics of Ngn3_{cm}, miR30Pdx1_{g-shRNA} and Pdx1_{cm} can be visualised in real time because miR30Pdx I_{g-shRNA} is linked to the expression of the blue-to-red medium fluorescent timer (mFT) and because the P_{CREm}-mediated dicistronic expression of Pdx1_{cm}-2A-MafA_{cm} can be linked to an isogenic P_{CREm}-driven EYFP expression plug-in (pSP24; P_{CREm}-EYFP-pA_{SV40}). During the co-induction of Ngn3_{cm} and miR30Pdx1_{g-shRNA} as well as miR30Pdx1_{g-shRNA}-mediated Pdx1_{g} knock-down at medium vanillic acid concentrations, the cells first fluoresce in blue and then simultaneously in blue and red as the mFT matures. After switching the culture to high vanillic acid and repressing P_{3VanO2}-driven Ngn3_{cm}, miR30Pdx1_{g-shRNA} and mFT de novo synthesis, all cells contain fully matured mFT and exclusively fluoresce in red. After the transition to P_{CREm} mediated Pdx1_{cm} and MafA_{cm} production, the cells express EYFP and fluoresce in yellow.
(iii) MafA induction profile (OFF-ON): Because P_{CREm} has been optimised for tightness and decreased vanillic acid sensitivity, MafA_{cm} is repressed at low and medium vanillic acid concentrations, as is required for the early phase of the differentiation process when Ngn3_{cm} is induced and Pdx1_{g} is repressed (day 4; Fig. 2c). At high vanillic acid levels, MafA_{cm} is co-cistronically expressed with Pdx1_{cm} by P_{CREm}; therefore, the time-delayed production onset of MafA_{cm} matches that of Pdx1_{cm} (OFF-ON; Fig. 2c). MafA_{cm} triggers a positive feedback loop by activating Pdx1_{g} expression from its genomic promoter, which results in sustained high-level MafA_{g} and Pdx1_{g} expression that compensates for the initial lower-level induction by P_{CREm} (day 11; Fig. 2c,g,h).

### Example 3 - Characterization of pancreatic beta-like cells programmed by the differentiation - control network

The phenotype of pancreatic beta-like cells produced from hIPSCs using the differentiation - control network were compared to the phenotype of cells generated by methods as described in Pagliuca FW, et al. Cell 159, 428-439 (2014); Rezanina A, et al (2014), Nat Biotechnol 32, 1121-1133; and Russ HA, et al. EMBO J 34, 1759-1772 (2015) by incorporating a modified growth factor/chemical based culture method that included Alk5 inhibitor (Alk5i) to prevent dedifferentiation and thyroid hormone T3 (triiodothyronine) to enhance the maturation of pancreatic beta-like cells. Following the execution of differentiation program (day 11), insulin positive network-engineered cells and insulin-positive control cells were sorted and RNA profiling of key beta cell specific genes was performed relative to human pancreatic islets. Expression profiling was done for the following: ATP-binding cassette transporter sub-family C member 8 (Abcc8); Acyl-CoA oxidase 2 (Acox2); voltage-dependent, L type, alpha 1D subunit (Cacna1D); Chromogranin B (Chgb); Cytokeratin-19 (Ck19); Dipeptidyl-peptidase 4 (Dpp4); Forkhead box protein A1 (FoxA1); Frizzled 2 (Fzd2); Glucagon receptor (Gcgr); Glucokinase (Gck); Glis family zinc finger 3 (Glis3); Glucose transporter 2 (Glut2); Glucose-6-phosphatase 2 (G6pc2); Islet amyloid polypeptide (Iapp); Iroquois homeobox 2 (Irx2); Potassium channel, subfamily K, member 1/3 (Kcnk1/3,); Potassium inwardly-rectifying channel, subfamily J, member 11 (Kcnj11); V-maf musculoaponeurotic fibrosarcoma oncogene homologue A/B (MafA/B); Matrix metalloproteinase 2 (Mmp2); Motor neuron and pancreas homeobox 1 (Mnx1); Neurogenic differentiation factor 1 (NeuroD1); NK6 homeobox 1 (Nkx6.1); Onecut homeobox 2 (Onecut2); Paired box gene 4 (Pax4); Proprotein convertase ½ (Pcsk1/2); Surfactant protein D (Sftpd); Pancreatic and duodenal homeobox 1 (Pdxl); Solute carrier family 30, member 8; Snap25,Synaptosomal-associated protein (Slc30a8); (Syntaxin-1A), Stxbp1, Syntaxin binding protein 1 (Stx1A); Synaptotagmin-4 (Syt4); and Urocortin 3 (Ucn3).

The differentiation-control network engineered cells had similar expression level of (i) transcription factors MafA, NeuroD, Pax4 and Pdxl to human pancreatic islets with NeuroD, Pax4 and Pdxl being expressed at a lower level in cells derived using growth factor/chemical-based differentiation technique (Fig. 3a) (ii) Glucose-processing factors such as Gck (Glucokinase), G6pc2 (glucose-6-phosphatase 2) were expressed at high levels as was the insulin-processing factor Pcsk1 (prohormone convertase 1) compared to the cells derived using growth factor/chemical-based differentiation technique (Fig. 3b). (iii) Expression levels for the channels essential for the secretion of insulin was similar between the differentiation-control network and the cells derived using growth factor/chemical-based differentiation technique (Fig. 3c). (iv) Insulin expression was higher in differentiation-control network-engineered cells as compared to the cells derived using growth factor/chemical-based differentiation technique although the expression level in human pancreatic islets was still higher (Fig. 3d). The expression of somatostatin, glucagon was much lower as compared to the cells derived using growth factor/chemical-based differentiation technique (Fig. 3d). (v) Markers indicative of immature cells in the pancreas such as Ck19 (Cytokeratin 19), Dpp4 (Dipeptidyl peptidase 4) and Acox2 (Acyl-CoA oxidase 2) were expressed at lower levels in differentiation-control network-engineered cells whereas FoxA1 (Forkhead box A1) and Gcgr (Glucagon receptor) were expressed at a higher level (Fig. 3e). Control experiments validated that the transcription factors NeuroD1 (induced by Ngn3), Pdxl and MafA synergistically activate the insulin promoter (Fig. 17) and that neither MOR9-1 expression nor an artificially induced cAMP surge impacted insulin transcription (Fig. 18). FACS analysis revealed that ∼70% of differentiation-control network positive cells were also C-peptide positive while less than 10% were positive for glucagon and somatostatin (Fig. 4a). Electron microscopy imaging of engineered pancreatic beta-like cells showed the typical insulin containing vesicles that are found in mature beta cells (Fig. 4b). The dynamics of glucose-sensitive insulin secretion expressed by C-peptide release were similar in the engineered pancreatic beta-like cells as compared to human pancreatic islets (Fig. 4c). Growth factor/chemical-based differentiation technique derived control cells showed poor glucose-sensitive insulin secretion (Fig. 4c). These findings confirmed that the differentiation -control network was able to drive hIPSC-derived human pancreatic progenitors specifically into glucose-sensitive insulin-secreting pancreatic beta-like cells.

While there are shown and described presently preferred embodiments of the invention, it is to be understood that the invention is not limited thereto but may be otherwise variously embodied and practiced within the scope of the following claims. Since numerous modifications and alternative embodiments of the present invention will be readily apparent to those skilled in the art, this description is to be construed as illustrative only and is for the purpose of teaching those skilled in the art the best mode for carrying out the present invention.

## Claims

1. An expression system comprising
a first receptor molecule for a first ligand, and
a second receptor molecule for a second ligand, wherein said second receptor molecule is a transcription factor,
wherein binding of the first ligand to said first receptor molecule controls expression of one or more first expression cassettes via activation of an intracellular signaling cascade, wherein said one or more first expression cassettes encode said second receptor molecule and binding of the first ligand to the first receptor molecule thereby activates expression of the second receptor molecule;
wherein binding of the second ligand to said second receptor molecule represses expression of one or more second expression cassettes;
wherein said first and said second ligand are the same; and
wherein said first and second receptor molecules have opposing responsiveness to said ligand.

2. The expression system of claim 1, wherein the first receptor molecule is expressed on the cell surface and the second receptor molecule is intracellularly expressed.

3. The expression system of any one of the preceding claims, wherein said ligand is vanillic acid.

4. The expression system of any one of the preceding claims, wherein said first receptor molecule is MOR9-1.

5. The expression system of any one of the preceding claims, wherein said second receptor molecule is a vanillic acid-dependent transactivator.

6. The expression system of any one of the preceding claims, wherein said first expression cassettes encode Ngn3 and, wherein said second expression cassettes encode Pdx1 or MafA.

7. A plurality of isolated nucleic acids which in their entirety encode the expression system of any one of claims 1 to 6 or a single isolated nucleic acid encoding the expression system of any one of claims 1 to 6.

8. A host cell comprising the expression system of any one of claims 1 to 6 or the plurality of isolated nucleic acids or the single isolated nucleic acid of claim 7.

9. An in vitro method of differentiating a cell, comprising the steps of
a. Introducing the expression system of any one of claims 1 to 6 into said cell;
b. Adding varying doses of one or more ligands to said cell, thereby triggering the sequential expression of two or more genes of interest and altering the phenotype of said cell.

## Patentansprüche

1. Expressionssystem, umfassend
ein erstes Rezeptormolekül für einen ersten Liganden, und
ein zweites Rezeptormolekül für einen zweiten Liganden, wobei das zweite Rezeptormolekül ein Transkriptionsfaktor ist,
wobei das Binden des ersten Liganden an das erste Rezeptormolekül die Expression einer oder mehrerer ersten Expressionskassetten über die Aktivierung einer intrazellulären Signalkaskade steuert, wobei die eine oder die mehreren ersten Expressionskassetten für das zweite Rezeptormolekül kodieren und wodurch die Bindung des ersten Liganden an das erste Rezeptormolekül, die Expression des zweiten Rezeptormoleküls aktiviert;
wobei die Bindung des zweiten Liganden an das zweite Rezeptormolekül die Expression einer oder mehrerer zweiten Expressionskassetten unterdrückt;
wobei der erste und der zweite Ligand gleich sind; und
wobei das erste und das zweite Rezeptormolekül eine entgegengesetzte Reaktion bezüglich des Liganden aufweisen.

2. Expressionssystem nach Anspruch 1, wobei das erste Rezeptormolekül an der Zelloberfläche exprimiert wird und das zweite Rezeptormolekül intrazellulär exprimiert wird.

3. Expressionssystem nach einem der vorhergehenden Ansprüche, wobei der Ligand Vanillinsäure ist.

4. Expressionssystem nach einem der vorhergehenden Ansprüche, wobei das erste Rezeptormolekül MOR9-1 ist.

5. Expressionssystem nach einem der vorhergehenden Ansprüche, wobei das zweite Rezeptormolekül ein Vanillinsäure-abhängiger Transaktivator ist.

6. Expressionssystem nach einem der vorhergehenden Ansprüche, wobei die ersten Expressionskassetten für Ngn3 kodieren und die zweiten Expressionskassetten für Pdxl oder MafA kodieren.

7. Mehrere isolierte Nukleinsäuren, die in ihrer Gesamtheit für das Expressionssystem nach einem der Ansprüche 1 bis 6 kodieren oder eine einzelne isolierte Nukleinsäure, die für das Expressionssystem nach einem der Ansprüche 1 bis 6 kodiert.

8. Wirtszelle, umfassend das Expressionssystem nach einem der Ansprüche 1 bis 6 oder die mehreren isolierten Nukleinsäuren oder die einzelne isolierte Nukleinsäure nach Anspruch 7.

9. In-vitro-Verfahren zum Differenzieren einer Zelle, umfassend die Schritte:
a. Einführen des Expressionssystems nach einem der Ansprüche 1 bis 6 in die Zelle;
b. Hinzufügen unterschiedlicher Dosen eines oder mehrerer Liganden zu der Zelle, wodurch die sequentielle Expression von zwei oder mehr Genen von Interesse ausgelöst und der Phänotyp der Zelle verändert wird.

## Revendications

1. Système d'expression comprenant
une première molécule réceptrice pour un premier ligand, et
une seconde molécule réceptrice pour un second ligand, la seconde molécule réceptrice étant un facteur de transcription,
dans lequel la liaison du premier ligand à ladite première molécule réceptrice régule l'expression d'une ou plusieurs premières cassettes d'expression par activation d'une cascade de signalisations intracellulaires, lesdites une ou plusieurs premières cassettes d'expression codant pour ladite seconde molécule réceptrice, et la liaison du premier ligand à la première molécule réceptrice activant ainsi l'expression de la seconde molécule réceptrice ;
dans lequel la liaison du second liant à ladite seconde molécule réceptrice réprime l'expression d'une ou plusieurs secondes cassettes d'expression ;
dans lequel ledit premier et ledit second ligands sont les mêmes ; et
dans lequel lesdites première et seconde molécules réceptrices ont des réponses opposées audit ligand.

2. Système d'expression selon la revendication 1, dans lequel la première molécule réceptrice est exprimée sur la surface cellulaire et la seconde molécule réceptrice est exprimée au niveau intracellulaire.

3. Système d'expression selon l'une quelconque des revendications précédentes, dans lequel ledit ligand est l'acide vanillique.

4. Système d'expression selon l'une quelconque des revendications précédentes, dans lequel ladite première molécule réceptrice est MOR9-1.

5. Système d'expression selon l'une quelconque des revendications précédentes, dans lequel ladite seconde molécule réceptrice est un transactivateur dépendant de l'acide vanillique.

6. Système d'expression selon l'une quelconque des revendications précédentes, dans lequel lesdites premières cassettes d'expression codent pour Ngn3, et dans lequel lesdites secondes cassettes d'expression codent pour Pdxl ou MafA.

7. Pluralité d'acides nucléiques isolés qui, en leur totalité, codent pour le système d'expression selon l'une quelconque des revendications 1 à 6, ou acide nucléique isolé unique codant pour le système d'expression selon l'une quelconque des revendications 1 à 6.

8. Cellule hôte comprenant le système d'expression selon l'une quelconque des revendications 1 à 6 ou la pluralité d'acides nucléiques isolés ou l'acide nucléique isolé unique selon la revendication 7.

9. Méthode in vitro de différenciation d'une cellule, comprenant les étapes de
a. introduction du système d'expression selon l'une quelconque des revendications 1 à 6 dans ladite cellule ;
b. addition de différentes doses d'un ou plusieurs ligands à ladite cellule, en déclenchant ainsi l'expression séquentielle de deux gènes d'intérêt ou plus, et altérant le phénotype de ladite cellule.
